# EUROPEAN PATENT APPLICATION

(11) **EP 2 356 998 A1**
(43) Date of publication of application: **17.08.2011**
(21) Application number: 10153766.0
(22) Date of filing: 17.02.2010
(51) Int. Cl.: A61K 38/22, A61K 38/27, A61P 9/10

(54) **A pharmaceutical composition for treatment of thrombosis-related diseases comprising a fragment of prolactin (PRL)-growth hormone (GH) - placental lactogen (PL)-family protein**

(71) Applicant: Université de Liège, 4031 Angleur (BE)
(72) Inventor: Struman, Ingrid, 4000, Liège (BE); Martial, Joseph, 4000, Liège (BE); Bajou, Khalid, 4000, Liège (BE); D´Amico, Salvino, 4000, Liège (BE)
(74) Representative: Polypatent

(57) **Abstract**

The present invention refers to a pharmaceutical composition for use in the preventive and/or therapeutic treatment of thrombosis-related diseases or conditions relating to high level of PAI-1 expression, which composition is comprising
A) a fragment of a polypeptide or protein of prolactin (PRL)-growth hormone (GH) - placental lactogen (PL)-family and homologous derivatives thereof, which fragment comprises from 6 to 14 consecutive amino acid residues of the amino acid sequence having the following general formula (I):
X1-X2-X3-X4-X5-X6-X7-X8-X9-X10-X11-X12-X13-X14, wherein
X1 is an amino acid residue of: Leu, Phe;
X2 is an amino acid residue of: Leu;
X3 is an amino acid residue of: Arg, Ser;
X4 is an amino acid residue of: Ile, Leu;
X5 is an amino acid residue of: Ser, Ile;
X6 is an amino acid residue of: Leu, Val;
X7 is an amino acid residue of: Leu, Ser,
X8 is an amino acid residue of: Leu, Ile;
X9 is an amino acid residue of: Ile, Leu;
X10 is an amino acid residue of: Gln, Glu, Arg;
X11 is an amino acid residue of: Ser;
X12 is an amino acid residue of: Trp;
X13 is an amino acid residue of: Leu, Asn;
X14 is an amino acid residue of: Glu (SEQ ID NO: 11); or

B) a peptide or a recombinant protein comprising said peptide, wherein the peptide is from 6 to 50 amino acids in length and has the activity to inhibit plasminogen activator inhibitor I (PAI-1), said peptide comprising from 6 to 14 consecutive amino acid residues of the amino acid sequence of said general formula (I); or
C) a polynucleotide encoding said fragment of a polypeptide or protein of A) or said peptide or recombinant protein of B).

## Description

The present invention refers to therapeutics and a therapeutic method for improving the health of patients suffering from a consequence of thrombosis. In particular, the invention relates to 16-kDa N-terminal fragments of proteins of the prolactin/growth hormone family, and fragments thereof, for use in the treatment of patients suffering from thrombotic disorders. The present invention refers to a pharmaceutical composition for use in the preventive and/or therapeutic treatment of thrombosis-related diseases, which composition is comprising a fragment of a polypeptide/protein of prolactin (PRL)-growth hormone (GH) - placental lactogen (PL)-family, or a peptide or a recombinant protein comprising said peptide, wherein the peptide is between 6 and 50 amino acids in length, said fragment or peptide having the activity to inhibit plasminogen activator inhibitor 1 (PAl-1), thereby resulting in thrombolytic activity.

### Background of the invention

### Thrombosis and Haemostasis

The hemostatic process is a host defense mechanism to preserve the integrity of the closed high pressure circulatory system. This process must remain inactive but poised to immediately minimize extravasation of blood from the vasculature following tissue injury. When a blood vessel is injured, blood clot (thrombus) is formed from circulating platelets that are recruited to the site of injury; then blood coagulation, initiated by tissue factor, culminates in the generation of thrombin and fibrin. Such a process must be activatable within seconds of injury. After this process is activated, it remains critical to contain thrombus formation so that it is localized to the site of injury and to modulate thrombus size to be proportionate to the injury. Thus, there is a balance between molecules that initiate thrombus formation (fibrin, thrombin...) and the ones that dissolve the thrombus (plasmin, plasminogen activators...). Fibrin and plasmin are generated *in situ* from inactive precursors (fibrinogen and plasminogen) by respectively thrombin and the pasminogen activators (tPA and uPA, see further : "the fibrinolytic system"). When pathological processes overwhelm the regulatory mechanisms of haemostasis, excessive quantities of thrombin are formed, initiating thrombosis.

Thrombosis can occur anywhere in the body's bloodstream. There are two main types of thrombosis: venous and arterial thrombosis:
Venous thrombosis results from a blood clot that develops in a vein. One of the most common types of venous thrombosis is deep vein thrombosis (DVT), which is a blood clot in one of the deep veins of the body. Deep vein thrombosis commonly affects the leg veins and occasionally the veins of the arm. Sometimes a blood clot (or part of one) can come away from its original site and travel through the bloodstream. If this occurs, the clot can become lodged in another part of the body. This is known as an embolism. A blood clot that lodges in one of the lungs is called a pulmonary embolism.

Arterial thrombosis, which is a blood clot that develops in an artery, often occurs in arteries that supply the heart, resulting in a heart attack. It can also occur in the arteries of the brain, causing a stroke.The primary trigger of arterial thrombosis is the rupture of atherosclerotic plaque, which develops through the accumulation of lipid deposits and lipid-laden macrophages (foam cells) in the artery wall. The secretion of cytokines and growth factors by plaque cells, and the further deposition of extracellular matrix components, contribute to plaque progression, causing a narrowing of the arterial lumen (stenosis). The central core of the mature plaques can become necrotic, and neovascularization in the plaques can allow leakage of blood components and haemorrhage. Over time, the secretion of matrix-degrading proteases and cytokines by plaque cells can cause a thinning of the fibrous cap, which prevents contact between the blood and the pro-thrombotic material in the plaque. Ultimately, the cap can disintegrate, causing plaque erosion or rupture. The ensuing discharge of plaque debris and the release of tissue factor into the blood trigger the coagulation cascade and formation of a thrombus, which can block the artery and result in coronary syndromes, myocardial infarction, or stroke.

Acute arterial thrombosis is the proximal cause of most cases of myocardial infarction (heart attack) and of about 80% of strokes. Together, these two conditions constitute the most common cause of death in the developed world. Venous thromboembolism is the third leading cause of cardiovascular-associated death.

### The fibrinolytic system

The fibrinolytic system, also known as the "plasminogen activator system", plays an essential role in maintaining the integrity of the vascular system and in dissolving blood clots. The ultimate enzyme responsible for the degradation of fibrin fibres in blood clots is the proteinase plasmin. This proteinase is produced from an inactive precursor, plasminogen, which circulates at relatively high concentration in the plasma. The two main activators of plasminogen are *tissue-type plasminogen activator* (tPA) and *urokinase-type plasminogen activator* (uPA). The former is involved mainly in dissolving bloodstream fibrin, whereas the latter binds to the *cellular uPAR receptor* (uPAR), causing activation of cell-bound plasminogen. Thus, uPAR is involved primarily in pericellular proteolysis, during tissue remodelling and repair, cell migration, and tumor invasion for example. The fibrinolytic system is negatively regulated by proteinase inhibitors. An important inhibitor of plasmin is α2-antiplasmin. At the level of plasminogen activation, the system is regulated mainly by *plasminogen activator inhibitor-1* (PAl-1). Although the PAl-1 concentration is quite low in the bloodstream, its presence in platelets leads to a high local concentration in thrombi, suggesting an important role of PAI-1 in blood clot maintenance. The most recently identified player in the system is *thrombin-activatable fibrinolysis inhibitor* (TAFI). Although its mechanism of action is not yet fully understood, TAFl appears to reduce fibrinolysis by cleaving C-terminal lysines on partially degraded fibrin, thereby interfering with efficient plasminogen activation.

During thrombus formation, circulating prothrombin is activated by activated platelets, forming thrombin, the active clotting factor. Fibrinogen is converted to fibrin by the newly activated thrombin. Fibrin then forms the fibrin matrix. All this takes place while platelets are adhering and aggregating. Current drugs used to fight thrombosis target three main components of blood clot: platelets, thrombin, and fibrin and are respectively known as antiplatelet drugs, thrombolytics or fibrinolytics.

Antiplatelet drugs target platelet activation and aggregation. Aspirin has been used clinically for more than 40 years and is the most commonly used antiplatelet drug. More recent antiplatelets drugs are thienopyridines : inhibitors of the ADP receptor P2Y12 such as clopidogrel, prasugrel, and cangrelor or αllbβ3 integrin inhibitors such as abciximab and eptifibatide. Anticoagulants are used to treat or prevent a wide variety of conditions involving arterial or venous thrombosis. They are notably used to prevent venous thromboembolism and for long-term prevention of ischaemic stroke in patients with atrial fibrillation. The two main classes of anticoagulant drugs are vitamin K antagonists and heparin, which target multiple proteases in the coagulation cascade. As with anti-platelet drugs, the main side effect of anticoagulant therapy is bleeding.

Plasminogen accumulates in the fibrin matrix. Thrombolytic drugs promote the conversion of fibrin-bound plasminogen to plasmin, the rate-limiting step in thrombolysis. The thrombolytic process works best on recently formed thrombi. Older thrombi display extensive fibrin polymerization making them more resistant to thrombolysis. Time is thus important in thrombolytic therapy. The thrombolytic agents available today are serine proteases that work by converting plasminogen to the natural fibrinolytic agent plasmin. Plasmin lyses a clot by breaking down the fibrinogen and fibrin it contains. Tissue plasminogen activator (tPA) is a naturally occurring fibrinolytic agent found in vascular endothelial cells. It contributes to maintaining a balance between thrombolysis and thrombogenesis. It targets fibrin with high specificity and affinity. At the site of the thrombus, the binding of tPA and plasminogen to the fibrin surface induces a conformational change facilitating the conversion of plasminogen to plasmin and dissolving the clot.

Fibrinolytics, sometimes referred to as plasminogen activators, are divided into two categories. Fibrin-specific agents such as alteplase, reteplase, and tenecteplase produce limited plasminogen conversion in the absence of fibrin, whereas non-fibrin-specific agents such as streptokinase catalyze systemic fibrinolysis. Streptokinase is indicated for the treatment of acute myocardial infarction, acute massive pulmonary embolism, deep vein thrombosis, arterial thrombosis, and occluded arteriovenous cannulae. Streptokinase is not widely used in the United States but continues to be used elsewhere because of its lower cost. Alteplase is the only current lytic agent approved by the US Food and Drug Administration (FDA) for acute myocardial infarction, acute ischaemic stroke, massive pulmonary embolism, and occluded central venous access devices.

The success of treatment for acute thrombotic events with fibrinolytics depends crucially on the timing of intervention, with earlier intervention generally having a better outcome. For example, for acute myocardial infarction, fibrinolytic therapy seems to be beneficial for at least 12 hours after the onset of symptoms. By contrast, fibrinolytic therapy for stroke has proven beneficial only when used within 3 hours and can have the side effect of inducing brain haemorrhage. Therefore, researchers are focusing on strategies that protect the vasculature but have a lower incidence of brain haemorrhage than is induced by current fibrinolytic therapy.

### Pathologies associated with increased PAl-1 levels.

In some condition the level of PAl-1 in the body can increase considerably. For example, genetic polymorphism (4G/5G) in the promoter region of the PAI-1 gene has been correlated with high levels of PAI-1. Several studies have shown that this polymorphism is correlated with increased risk for cardiovascular diseases, ischemic strokes or thromboembolism. Thus finding molecule that could inhibits PAI-1 activity might be beneficial for treating patient suffering from an excessive PAI-1 level.

Although the available anti-thrombotic agents have proven significant clinical benefit, residual morbidity and mortality remain high, and their utility is always counterbalanced by the risk of bleeding complications. There was a desire to develop novel anti-thrombotic agents with a more favourable safety profile, better efficacy, and rapid onset and cessation of action in acute events.

The object of the present invention was to provide new compounds for the treatment of thrombosis-related diseases or for the treatment of conditions relating to high level of PAl-1 expression. Further, the object of the present invention was to provide a natural inhibitor of PAl-1.

Therefore, the present invention provides a pharmaceutical composition for use in the preventive and/or therapeutic treatment of thrombosis-related diseases or conditions relating to high level of PAl-1 expression, which composition is comprising:
A) a fragment of a polypeptide/protein of prolactin (PRL)-growth hormone (GH) - placental lactogen (PL)-family and homologous derivatives thereof, which fragment comprises from 6 to 14 consecutive amino acid residues of the amino acid sequence having the following general formula (I):
   X1-X2-X3-X4-X5-X6-X7-X8-X9-X10-X11-X12-X13-X14, wherein
   X1 is an amino acid residue of: Leu, Phe;
   X2 is an amino acid residue of: Leu;
   X3 is an amino acid residue of: Arg, Ser;
   X4 is an amino acid residue of: Ile, Leu;
   X5 is an amino acid residue of: Ser, Ile;
   X6 is an amino acid residue of: Leu, Val;
   X7 is an amino acid residue of: Leu, Ser,
   X8 is an amino acid residue of: Leu, Ile;
   X9 is an amino acid residue of: Ile, Leu;
   X10 is an amino acid residue of: Gln, Glu, Arg;
   X11 is an amino acid residue of: Ser;
   X12 is an amino acid residue of: Trp;
   X13 is an amino acid residue of: Leu, Asn;
   X14 is an amino acid residue of: Glu, (SEQ ID NO: 11); or
B) a peptide or a recombinant protein comprising said peptide, wherein the peptide is from 6 to 50 amino acids in length and has the activity to inhibit plasminogen activator inhibitor 1 (PAl-1), said peptide comprising from 6 to 14 consecutive amino acid residues of the amino acid sequence of said general formula (I);
C) a polynucleotide encoding said fragment of a polypeptide or protein of A) or said peptide or recombinant protein of B).

In a preferred embodiment the peptide of B) is from 6 to 45, further preferred from 6 to 40, still further preferred from 6 to 30, even further preferred from 6 to 20, more preferred from 6 to 15, particularly preferred from 6 to 12, more particularly preferred from 6 to 10 and most preferred 6, 7, 8 or 9 amino acids in length and has the activity to inhibit plasminogen activator inhibitor 1 (PAl-1).

In a preferred embodiment of the pharmaceutical composition said recombinant protein comprising said peptide represents a fusion protein wherein said peptide is fused N-terminally or C-terminally to a carrier protein or wherein said peptide is inserted into a carrier protein sequence in order to form a hybrid protein.

In one embodiment the fragment of a polypeptide/protein of prolactin (PRL)-growth hormone (GH) - placental lactogen (PL)-family is the 14-16K N-terminal fragment cleaved from the full-length polypeptide or protein of prolactin (PRL)-growth hormone (GH) - placental lactogen (PL)-family, which 14-16k fragment inhibit plasminogen activator inhibitor 1 (PAI-1). In another embodiment the respective 14-16K N-terminal fragment is expressed as such from a polynucleotide encoding this 14-16K N-terminal fragment.

These 14-16 kD fragments having the activity of inhibiting plasminogen activator inhibitor 1 (PAl-1) are those having the sequences SEQ ID NOs: 2, 3, 4 (hPRL fragments), SEQ ID NO: 6 (hPL fragment), SEQ ID NO: 8 (hGH fragment) and SEQ ID NO: 10 (hGH-v fragment) shown in the sequence listing. Proteins and polypeptides which are homologous to said sequences (i.e. homologous derivatives), e.g. having identity score of at least 80%, at least 90%, at least 95%, at leat 99% to those 14-16 kD fragments may also be used in the pharmaceutical composition of the present invention as they have the activity to inhibit plasminogen activator inhibitor 1 (PAl-1), which activity is attributed to the X1-X14 amino acid sequence or fragments thereof.

Alternatively, homologous derivatives of the 14-16K N-terminal fragment of prolactin (PRL), growth hormone (GH), growth hormone variant (GH-V) and placental lactogen (PL) are defined to differ from those in 1 to 40, preferably in 1 to 20, further preferred in 1 to 10 and most preferred in 1, 2, 3, 4 or 5 amino acid residue positions.

Any derivative of 14-16K N-terminal fragment of a prolactin (PRL)-growth hormone (GH) - placental lactogen (PL)-family polypeptide or protein is suitable as long as said fragment comprises from 6 to 14 consecutive amino acid residues of the amino acid sequence of said general formula (I).

The present inventors discovered that 14-16K fragments of the prolactin and growth hormone family and specific small peptides thereof inhibit the ability of PAI-1 to block tPA and uPA. The 14-16K fragments and specific small peptides thereof therefore may be useful to improve efficiency of tPA or other fibrinolytics. 14-16K fragments and specific small peptides thereof could be used in combination with fibrinolytics or alone to inhibit endogenous PAl-1. Another possibility is to use the 14-16K fragments or said specific small peptides thereof in patients who suffer from high level of PAl-1, which is related or not to thrombosis.

Further preferred, the amino acid sequence X1-X14 is having at least 71%, preferably at least 78%, more preferably at least 85% and most preferred at least 92% identity to one of the following sequences:
a) Leu Leu Arg Ile Ser Leu Leu Leu Ile Gln Ser Trp Leu Glu (SEQ ID NO: 12);
b) Leu Leu Arg Ile Ser Leu Leu Leu Ile Glu Ser Trp Leu Glu (SEQ ID NO: 13);
c) Phe Leu Ser Leu Ile Val Ser Ile Leu Arg Ser Trp Asn Glu (SEQ ID NO: 14); wherein the replaced amino acid residues are replaced by homologous amino acid residues.

In another preferred embodiment, at least 10, at least 11, at least 12, at least 13 or all 14 amino acid residue positions of the amino acid sequence X1-X14 are identical to those of any one of the following sequences:
a) Leu Leu Arg Ile Ser Leu Leu Leu Ile Gln Ser Trp Leu Glu (SEQ ID NO: 12);
b) Leu Leu Arg Ile Ser Leu Leu Leu Ile Glu Ser Trp Leu Glu (SEQ ID NO: 13);
c) Phe Leu Ser Leu Ile Val Ser Ile Leu Arg Ser Trp Asn Glu (SEQ ID NO: 14); wherein the replaced amino acid residues are replaced by homologous amino acid residues.

In a further preferred embodiment of the pharmaceutical composition the amino acid sequence X1-X14 is represented by any one of the following sequences:
a) Leu Leu Arg Ile Ser Leu Leu Leu Ile Gln Ser Trp Leu Glu (SEQ ID NO: 12);
b) Leu Leu Arg Ile Ser Leu Leu Leu Ile Glu Ser Trp Leu Glu (SEQ ID NO: 13);
c) Phe Leu Ser Leu Ile Val Ser Ile Leu Arg Ser Trp Asn Glu (SEQ ID NO: 14).

In a still further preferred embodiment of the pharmaceutical composition the peptide of B) comprises or consists of an amino acid sequence selected from any 6mer, 7mer, 8mer, 9mer 10mer, 11 mer, 12mer, 13mer fragment of the amino acid sequence selected from the group consisting of
a) the general formula (I):
   X1-X2-X3-X4-X5-X6-X7-X8-X9-X10-X11-X12-X13-X14, wherein the amino acid residues X1 to X14 are as defined above (SEQ ID NO: 11);
b) Leu Leu Arg Ile Ser Leu Leu Leu Ile Gln Ser Trp Leu Glu (SEQ ID NO: 12);
c) Leu Leu Arg Ile Ser Leu Leu Leu Ile Glu Ser Trp Leu Glu (SEQ ID NO: 13);
d) Phe Leu Ser Leu Ile Val Ser Ile Leu Arg Ser Trp Asn Glu (SEQ ID NO: 14) ; or wherein the peptide comprises or consists of the respective 14mer amino acid sequence.

In a particularly preferred embodiment of the pharmaceutical composition, the peptide of B) comprises or consists of an amino acid sequence selected from the following:
a) Leu Leu Arg Ile Ser Leu (SEQ ID NO: 15);
b) Arg Ile Ser Leu Leu Leu (SEQ ID NO: 16);
c) Ser Leu Leu Leu Ile Gln (SEQ ID NO: 17);
d) Leu Leu Ile Gln Ser Trp (SEQ ID NO: 18);
e) Ile Gln Ser Trp Leu Glu (SEQ ID NO: 19);
f) Ser Leu Leu Leu Ile Glu (SEQ ID NO: 20);
g) Leu Leu Ile Glu Ser Trp (SEQ ID NO: 21);
h) Ile Glu Ser Trp Leu Glu (SEQ ID NO: 22);
i) Phe Leu Ser Leu Ile Val (SEQ ID NO: 23);
j) Ser Leu Ile Val Ser Ile (SEQ ID NO: 24);
k) Ile Val Ser Ile Leu Arg (SEQ ID NO: 25);
l) Ser Ile Leu Arg Ser Trp (SEQ ID NO: 26);
m) Leu Arg Ser Trp Asn Glu (SEQ ID NO: 27).

The present invention also provides the use of a compound for the manufacture of a medicament for use in the preventive and/or therapeutic treatment of thrombosis-related diseases or conditions relating to high level of PAl-1 expression, which compound is selected from the following:
A) a fragment of a polypeptide/protein of prolactin (PRL)-growth hormone (GH) - placental lactogen (PL)-family and homologous derivatives thereof, which fragment comprises from 6 to 14 consecutive amino acid residues of the amino acid sequence having the following general formula (I):
   X1-X2-X3-X4-X5-X6-X7-X8-X9-X10-X11-X12-X13-X14, wherein the amino acid residues X1 to X14 are as defined above (SEQ ID NO: 11);
B) a peptide or a recombinant protein comprising said peptide, wherein the peptide is from 6 to 50 amino acids in length and has the activity to inhibit plasminogen activator inhibitor 1 (PAl-1), said peptide comprising from 6 to 14 consecutive amino acid residues of the amino acid sequence of said general formula (I);
C) a polynucleotide encoding said fragment of a polypeptide or protein of A) or said peptide or recombinant protein of B).

In a preferred embodiment the peptide is from 6 to 45, further preferred from 6 to 40, still further preferred from 6 to 30, even further preferred from 6 to 20, more preferred from 6 to 15, particularly preferred from 6 to 12, more particularly preferred from 6 to 10 and most preferred 6, 7, 8 or 9 amino acids in length and has the activity to inhibit plasminogen activator inhibitor 1 (PAl-1).

The present invention further provides a method for the preventive and/or therapeutic treatment of thrombosis-related diseases or conditions relating to high level of PAI-1 expression, comprising the administration of a pharmaceutically active amount of the compound which is defined as follows:
A) a fragment of a polypeptide/protein of prolactin (PRL)-growth hormone (GH) - placental lactogen (PL)-family and homologous derivatives thereof, which fragment comprises from 6 to 14 consecutive amino acid residues of the amino acid sequence having the following general formula (I):
   X1-X2-X3-X4-X5-X6-X7-X8-X9-X10-X11-X12-X13-X14, wherein the amino acid residues X1 to X14 are as defined above (SEQ ID NO: 11);
B) a peptide or a recombinant protein comprising said peptide, wherein the peptide is from 6 to 50 amino acids in length and has the activity to inhibit plasminogen activator inhibitor 1 (PAl-1), said peptide comprising from 6 to 14 consecutive amino acid residues of the amino acid sequence of said general formula (I);
C) a polynucleotide encoding said fragment of a polypeptide or protein of A) or said peptide or recombinant protein of B).

In preferred embodiments of the pharmaceutical composition, the use or the method according to the present invention the thrombosis-related disease is selected from venous thrombosis, arterial thrombosis, acute myocardial infarction, stroke.

In further preferred embodiments the peptide is from 6 to 45, further preferred from 6 to 40, still further preferred from 6 to 30, even further preferred from 6 to 20, more preferred from 6 to 15, particularly preferred from 6 to 12, more particularly preferred from 6 to 10 and most preferred 6, 7, 8 or 9 amino acids in length and has the activity to inhibit plasminogen activator inhibitor 1 (PAl-1).

Further, the present invention also provides a peptide or a recombinant protein comprising said peptide, wherein the peptide is from 6 to 50 amino acids in length and comprises from 6 to 10 consecutive amino acid residues of the amino acid sequence of the amino acid sequence of the general formula (I):
X1-X2-X3-X4-X5-X6-X7-X8-X9-X10-X11-X12-X13-X14, wherein the amino acid residues X1 to X14 are defined as follows:
   X1 is an amino acid residue of: Leu, Phe;
   X2 is an amino acid residue of: Leu;
   X3 is an amino acid residue of: Arg, Ser;
   X4 is an amino acid residue of: Ile, Leu;
   X5 is an amino acid residue of: Ser, Ile;
   X6 is an amino acid residue of: Leu, Val;
   X7 is an amino acid residue of: Leu, Ser,
   X8 is an amino acid residue of: Leu, Ile;
   X9 is an amino acid residue of: Ile, Leu;
   X10 is an amino acid residue of: Gln, Glu, Arg;
   X11 is an amino acid residue of: Ser;
   X12 is an amino acid residue of: Trp;
   X13 is an amino acid residue of: Leu, Asn;
   X14 is an amino acid residue of: Glu (SEQ ID NO: 11);
with the proviso that said peptide or said recombinant protein does not comprise or consist of any one of the following amino acid sequences:
a) from 11 to 14 consecutive amino acid residues of the amino acid sequence of the amino acid sequence of the general formula (I);
b) wherein the amino acid sequence X1-X14 of the peptide is having at least 70% identity to any one of the following sequences:
   i) Leu Leu Arg Ile Ser Leu Leu Leu Ile Gln Ser Trp Leu Glu (SEQ ID NO: 12);
   ii) Leu Leu Arg Ile Ser Leu Leu Leu Ile Glu Ser Trp Leu Glu (SEQ ID NO: 13);
   iii) Phe Leu Ser Leu Ile Val Ser Ile Leu Arg Ser Trp Asn Glu (SEQ ID NO: 14);
c) Leu Leu Ile Gln Ser Trp Leu Glu Pro Val Gln Phe Leu Arg Ser Val Phe Ala Asn Ser (SEQ ID NO: 28).

For example, the peptide or said recombinant protein of the present invention does also not comprise or consist of any one of the following amino acid sequences
d) Ser Asn Leu Glu Leu Leu Arg Ile Ser Leu Leu Leu Ile Gln Ser Trp Leu Glu Pro Val (SEQ ID NO: 29);
e) Asn Gln Lys Asp Phe Leu Ser Leu Ile Val Ser Ile Leu Arg Ser Trp Asn Glu Pro Leu Tyr His Leu Val Thr Glu Val Arg Gly Met Gln Glu Ala Pro Glu Ala Ile (SEQ ID NO: 30);
f) Asn Leu Glu Leu Leu Arg Ile Ser Leu Leu Leu Ile Gln Ser Trp Leu Glu Pro Val Gln Phe Leu Arg Ser Val Phe Ala Asn Ser (SEQ ID NO: 31);
g) Leu Ser Gln Ile Leu Ser Ser Leu Ile Gln Ser Trp Leu Glu (SEQ ID NO: 32);
h) Phe Asn Ser Leu Ile Ser Ser Ile Leu Arg Val Trp Leu Glu (SEQ ID NO: 33);
j) Ile Ser Leu Leu Leu Ile Gln Ser Trp Leu Glu Pro Val Gln Phe Leu Arg (SEQ ID NO: 34).

In a preferred embodiment the peptide is from 6 to 45, further preferred from 6 to 40, still further preferred from 6 to 30, even further preferred from 6 to 20, more preferred from 6 to 15, particularly preferred from 6 to 12, more particularly preferred from 6 to 10 and most preferred 6, 7, 8 or 9 amino acids in length and has the activity to inhibit plasminogen activator inhibitor 1 (PAl-1).

In a further preferred embodiment of the peptide or the recombinant protein comprising said peptide, the peptide comprises from 6 to 9, 6 to 8, 6 to 7, or 6 consecutive amino acid residues of the amino acid sequence of the amino acid sequence of the general formula (I):
X1-X2-X3-X4-X5-X6-X7-X8-X9-X10-X11 -X12-X13-X14, wherein the amino acid residues X1 to X14 are defined as above.

That is in such embodiment of said peptide or said recombinant protein comprising said peptide according to the present invention, the peptide comprises or consists of an amino acid sequence selected from a 6mer, 7mer, 8mer, 9mer, 10mer, fragment of the amino acid sequence of the general formula (I):
X1-X2-X3-X4-X5-X6-X7-X8-X9-X10-X11-X12-X13-X14, wherein the amino acid residues X1 to X14 are as defined above.

In a still further preferred embodiment of said peptide or said recombinant protein comprising said peptide according to the present invention, the peptide comprises or consists of an amino acid sequence selected from the following:
a) Leu Leu Arg Ile Ser Leu (SEQ ID NO: 15);
b) Arg Ile Ser Leu Leu Leu (SEQ ID NO: 16);
c) Ser Leu Leu Leu Ile Gln (SEQ ID NO: 17);
d) Leu Leu Ile Gln Ser Trp (SEQ ID NO: 18);
e) Ile Gln Ser Trp Leu Glu (SEQ ID NO: 19);
f) Ser Leu Leu Leu Ile Glu (SEQ ID NO: 20);
g) Leu Leu Ile Glu Ser Trp (SEQ ID NO: 21);
h) Ile Glu Ser Trp Leu Glu (SEQ ID NO: 22);
i) Phe Leu Ser Leu Ile Val (SEQ ID NO: 23);
j) Ser Leu Ile Val Ser Ile (SEQ ID NO: 24);
k) Ile Val Ser Ile Leu Arg (SEQ ID NO: 25);
l) Ser Ile Leu Arg Ser Trp (SEQ ID NO: 26);
m) Leu Arg Ser Trp Asn Glu (SEQ ID NO:27).

The present invention also provides a polynucleotide encoding the peptide or recombinant protein of the present invention and a transformant comprising the polynucleotide of the present invention. Said peptide or said recombinant protein comprising said peptide is also provided in a pharmaceutical composition.

### Description of the drawings

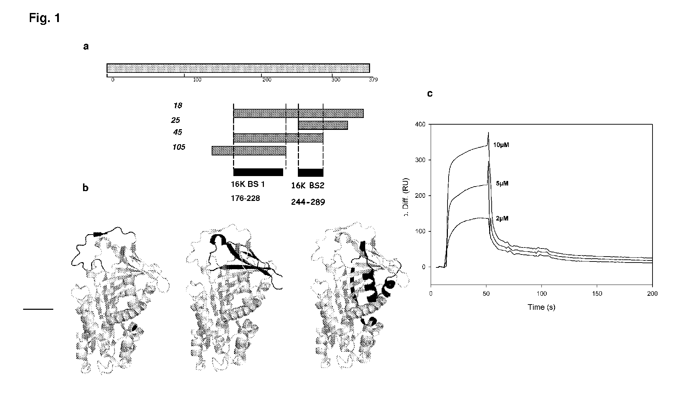

**Figure 1** shows that 16K hPRL interacts with PAI-1. (a) Interaction of 16K hPRL and PAI-1 in the yeast two-hybrid system. A HUVEC cDNA library has been screened using 16K hPRL cDNA cloned in the Gal-4 bait vector. 4 clones encoding several region of PAl-1 have been isolated during this screen and correspond to the aminoacids sequences K176/V364 (clone18), E244/E313 (clone28), G174/K289 (clone45), W140/P228 (clone105). Dark boxes: two regions in common in at least 3 clones are named BS16K1 and BS16K2. (b) Location of the 16K putative binding sites on the PAI-1 3-D structure. Highlited in dark: BS16K1 (middle) and BS16K2 (right) are in proximity of the RCL (left). (c) Surface plasmon resonance analysis of the interaction between PAI-1 and 16K hPRL. Analysis of binding kinetics of interaction between 16K hPRL and immobilized PAl-1. Representative dose-response sensorgram for 16K hPRL.

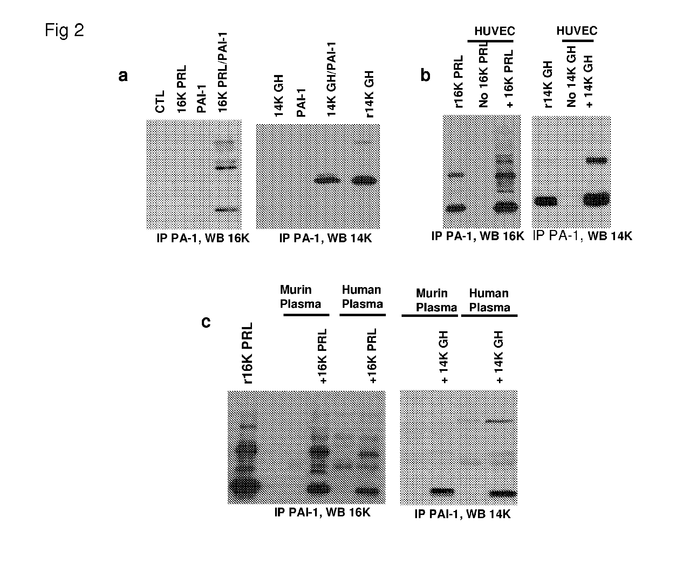

**Figure 2** shows that 16K PRL or 14K GH associate with recombinant or endogenous PAl-1. (**a**) Western blot (WB) analysis of 16K PRL (left) and 14K GH (rigth) after co-immunoprecipitation (IP) of recombinant 16K PRL or 14K GH and human PAl-1. For all experiment, a monoclonal anti-PAl-1 (clone 33H1 F7) was used for IP. r14K GH and r16K PRL: recombinant proteins controls loaded on gels, no IP performed. CTL: control beads, no proteins. (**b**) Western blot analysis of 16K PRL (left) and 14K GH (right) after IP of endogenous PAl-1 in conditioned medium of HUVEC in the presence or in the absence of added 16K PRL or 14K GH. (**c**) Western blot analysis of 16K PRL (left) and 14K GH (right) after IP of PAl-1 in human or murin plasma in the presence or in the absence of added recombinant 16K PRL or 14K GH.

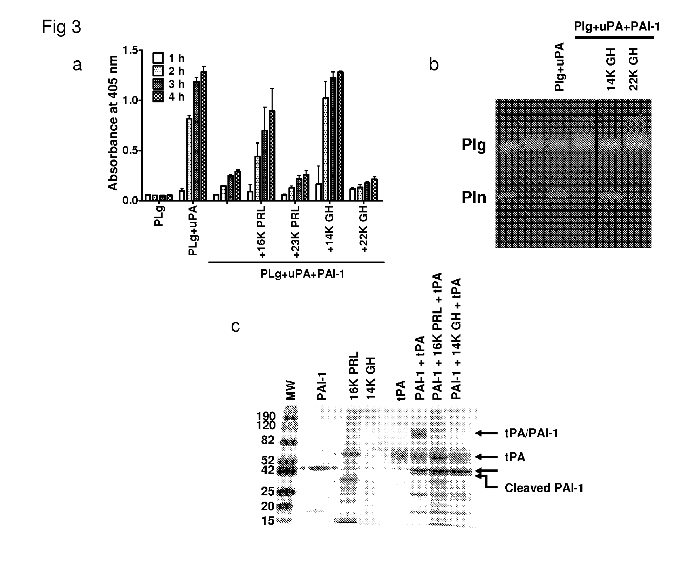

**Figure 3** shows the alteration of physiological function of PAl-1 in the presence of 16K PRL and 14K GH. (**a**) Proteolytic activity of uPA on plasminogen (Plg) in different conditions was determined by colorimetric assay (absorbance at 405nm) as described in Methods. The inhibition of plasminogen activation by PAI-1 in the presence of 16K PRL, 23K PRL, 14K GH or 22K GH was determined 1, 2 , 3 and 4 hours after addition of a colorimetric substrate. The data represent the means ± SD of duplicate samples and experiment was repeated at least three times. (**b**) Casein zymography showing Plg and plasmin (Pln) generation by uPA in the presence of PAl-1 alone or in combination with 14K GH or 22K GH. (**c**) SDS-PAGE silver stained showing the complex formation between PAI-1 and tPA in the absence or in the presence of 16K PRL or 14K GH.

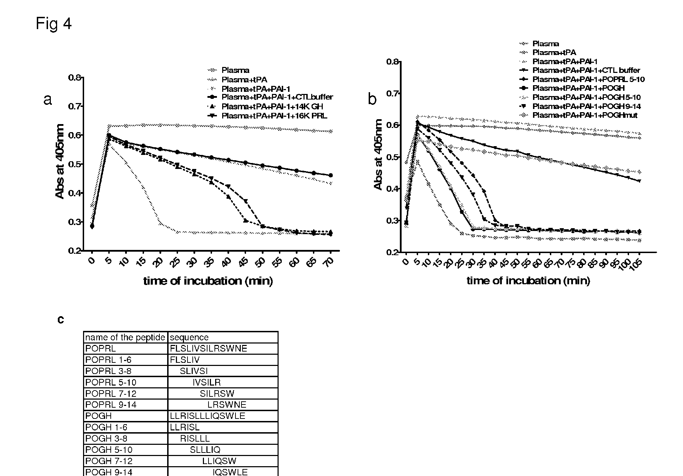

**Figure 4** shows that 16K PRL, 14K GH and derived shorter peptides, increase clot dissolution in human plasma. (**a**) Optical density at 405nm of a clot lysis assay. Clot were formed with pooled normal plasma and lysed by 180 pM of tPA. Inhibition of clot lysis by recombinant human PAI-1 is significantly reduced by 16K PRL and 14K GH. (**b**) Short peptides derived from 14K GH were added to human plasma in the presence of human PAI-1, and clot lysis was proportional to absorbance decrease. (**c**) List and sequence of the shorter peptides derived from the 16K PRL and 14K GH.

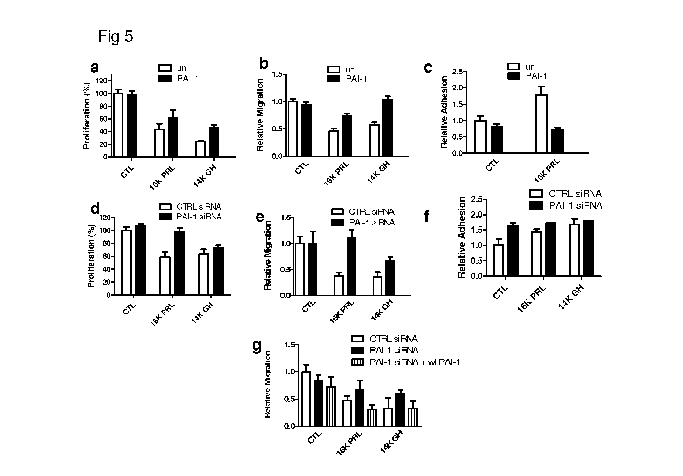

**Figure 5** shows that cell proliferation, cell adhesion and cell migration are altered in the presence of an excess or a reduced PAl-1 level. Endothelial cells were incubated with recombinant PAl-1 at 100nM, followed by 16K PRL or 14K GH treatment. Cell proliferation (**a**), adhesion (**b**) and migration (**c**) were analysed. 96 hours after transfection, PAl-1 siRNA- and CTL- siRNA- transfected cells were treated with 16K PRL or 14K GH, and cell proliferation (**d**), adhesion (**e**) and migration (**f**) were analyzed. (**g**) Cell migration after down regulation of PAl-1 by siRNA and addition of recombinant PAl-1 to the cell medium.

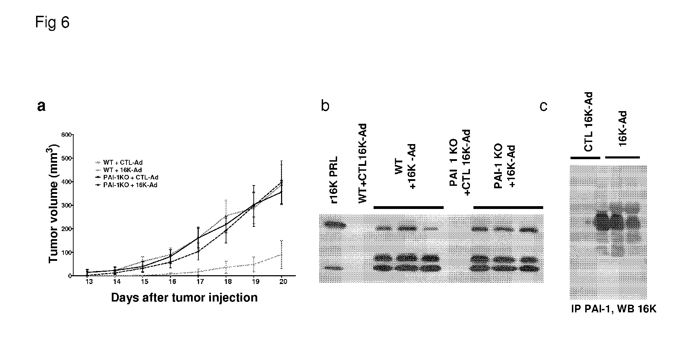

**Figure 6** shows the reduction of B16F10 tumor growth by 16K PRL is PAl-1 dependent. (**a**) Tumor volumes after subcutaneous injection of B16F10 in wild type (WT) or mice deficient for PAl-1 (PAI-1 KO) treated with adenovirus vectors allowing 16K PRL expressed (16K-Ad) or control (Null-Ad)(n = 7). Data are means ± SEM (**b**) Western blot analysis of 16K hPRL expression in plasma of different groups of mice. (**c**) Western blot analysis of 16K PRL from mice plasma injected with Null-Ad or 16K-Ad after IP of endogenous PAl-1.

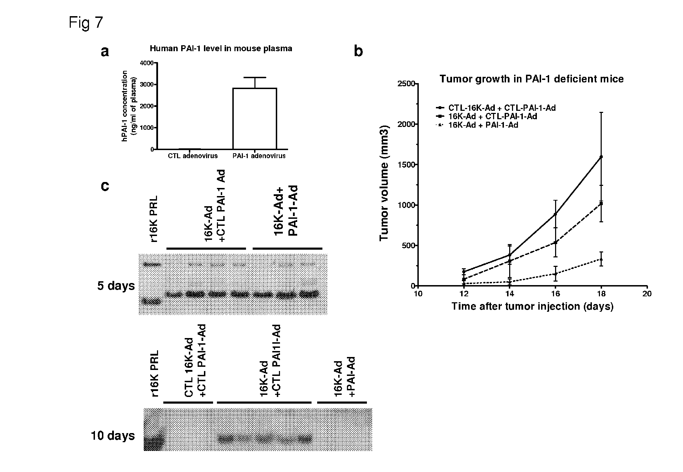

**Figure 7** shows systemic expression of human wild type PAl-1 in mice restores the anti-tumoral function of 16K PRL. (**a**) ELISA quantification of human PAl-1 in plasma of deficient mice, injected with CTL-Ad or PAl-1-Ad. (**b**) Tumor growth of B16F10 cells in PAl-1 deficient mice in the absence (CTL 16K-Ad) or in the presence of 16K hPRL (16K-Ad), and the restoration of human PAl-1 using adenovirus vectors (PAl-1-Ad) or the corresponding control adenovirus vector (CTL PAl1-Ad). (**c**) 16K hPRL expression determined by Western blot analysis in mice plasma 5 and 10 days after a second 16K hPRL adenovirus vector injection.

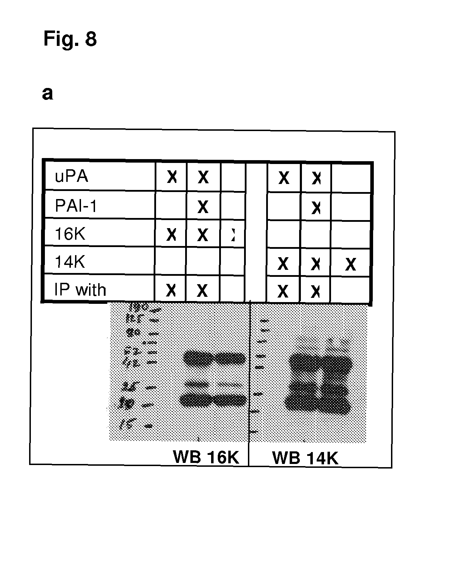

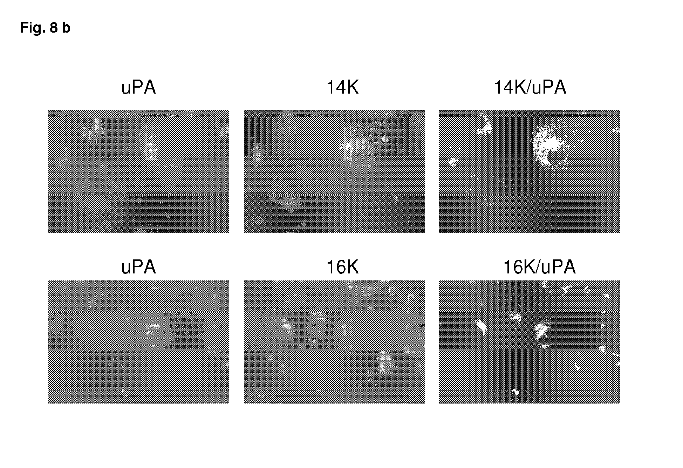

**Figure 8** shows that 16K PRL and 14K GH bind to PAl-1/uPA complex. (**a**) Western blot analysis of 16K PRL and 14K GH after their immunoprecipitation with uPA in the presence or in the absence of PAl-1. r14K GH and r16K PRL: recombinant proteins controls loaded on gels, no IP performed. (b) HUVEC were incubated with 14K GH or 16K PRL for 2 hours and then subjected to immunofluorescence analysis for uPA and 14K GH or 16K PRL Labellings appears in white for uPA (left panels), 14K GH or 16K PRL (middle panels). The right panels shows the colocalization of uPA and 14K GH or 16K PRL in white.

### Detailed description of the invention

The invention provides fragments of a polypeptide or protein of prolactin (PRL)-growth hormone (GH) - placental lactogen (PL)-family and homologous fragments thereof and small peptides thereof for preventive and/or therapeutic treatment of thrombosis-related diseases or conditions relating to high level of PAl-1 expression. These proteins and novel peptides are potent inhibitors of plasminogen activator inhibitor 1 (PAl-1).

As described in the prior art, the 16-kDa and 14-kDa fragments of the human prolactin/growth hormone (PRL/GH) family members are potent angiogenesis inhibitors. The molecular mechanisms by which these factors exert their antiangiogenic action is poorly understood, and so far the receptors involved remain unknown.

Using yeast two-hybrid technology, the present inventors have found that 16K PRL interacts with Plasminogen Activator Inhibitor type 1 (PAl-1). During the studies of the present invention this interaction of 16K PRL and 14K GH with PAl-1 was confirmed by surface plasmon resonance and further by co-immunoprecipitation with recombinant PAl-1 or endogenous PAl-1 from endothelial cell medium and from human and mouse plasma. Importantly, 16K PRL and 14K PRL inhibited the antiproteolytic function of PAl-1 and decreased or blocked formation of a complex between PAl-1 and plasminogen activators. In PAl-1-deficient mice, 16K PRL was unable to decrease B16F10 tumor growth, but the anti-tumor function of 16K PRL was restored after intravenous injection of an adenoviral vector expressing human PAl-1. This expression, furthermore, lead to clearance of 16K PRL from the bloodstream of PAl-1-deficient mice. Immunohistochemistry performed on human umbilical vein endothelial cells (HUVECs) showed that 16K PRL and 14K GH colocalized with uPA. The present data suggest that the PAl-1 molecule mediated the anti-tumor function of 16K PRL and 14K GH. Most importantly, 16K PRL, 14K GH, and certain isolated fragments thereof induced clot lysis *in vitro.* On the basis of all these data, the present invention proposes the use of these fragments, alone or in combination with fibrinolytics, to dissolve clots in the treatment of thrombotic disorders like acute myocardial infarction or stroke.

### The prolactin/growth hormone family and the 16-kDa fragments

Angiogenesis, i.e. the formation of new blood vessels from pre-existing ones, is a key event in tumor growth. It is also crucial for tumor metastasis, as blood vessels offer tumor cells an efficient route for leaving the primary site via the bloodstream. Angiogenesis is a complex process regulated by both activators and inhibitors of endothelial cell apoptosis, proliferation, migration, and organization. Many proteins, including growth factors, their receptors, extracellular matrix proteins, and matrix metalloproteinases, are involved in this process. Great efforts have been made to discover new antiangiogenic factors, and antiangiogenic therapy is viewed as an important approach to treating cancer.

In the prior art, recent studies of the prolactin/growth hormone family have revealed the antiangiogenic properties of the 16-kDa N-terminal fragment of prolactin (16K prolactin), obtained by cleavage of prolactin by cathepsin D (WO 2006/018418). Interestingly, the 16K fragments of all four members of the human PRL/GH family, i.e. GH-N, GH-V, PRL, and placental lactogen, have similar antiangiogenic properties, whereas the corresponding full-length hormones stimulate angiogenesis. More recently, shorter peptides have been identified, derived from these hormones that inhibit angiogenesis *in vivo* and *in vitro* (WO 2006/018418). Given the importance of angiogenesis in tumor progression and metastatic dissemination, the impact of 16K prolactin in models of physiological angiogenesis was evaluated (CAM assay, pathological retinopathy and several tumor models). Recently, 16K prolactin was also shown to considerably reduce the establishment of metastases in a lung metastasis model. In addition, excessive local generation of human 16K prolactin has been shown to cause postpartum cardiomyopathy. So far, the mechanism by which 16K prolactin inhibits angiogenesis is only partially elucidated. In the prior art it was demonstrated that 16K prolactin induces endothelial cell cycle arrest in G₀-G₁ and G₂-M states. This correlates with inhibition of MAPK activation induced by basic fibroblast growth factor (bFGF) and VEGF. In addition, it has been shown that 16K prolactin induces endothelial cell apoptosis and that nuclear factor κB (NF-κB) activation is required for this process. More recently, a microarray analysis performed on endothelial cell mRNAs after treatment with 16K prolactin revealed links between 16K prolactin and the immune system: 16K prolactin induces leukocyte adhesion to endothelial cells by activatiing NF-κB.

The examples presented in the present invention provide evidence that PAl-1 is the binding partner of 16K prolactin and 14K GH and that PAI-1 mediates the antiangiogenic properties of 16K prolactin and 14K GH *in vitro.* PAI-1 also mediates antitumoral activity of 16K Prolactin *in vivo.* In addition, the data obtained describe that 16K Prolactin and 14K GH or specific peptides thereof can improve clot dissolution and might be of potential use for treating patients suffering from thrombotic disorders.

### Definitions

The present invention is based on the finding that 14-16K N-terminal fragments cleaved from a polypeptide or protein of prolactin (PRL)-growth hormone (GH) - placental lactogen (PL)-family and smaller fragments thereof inhibit plasminogen activator inhibitor 1 (PAl-1).

As used herein the term 14-16 kD N-terminal fragment of a polypeptide or protein of prolactin (PRL)-growth hormone (GH) - placental lactogen (PL)-family means a fragment of about 14-16 kD ("16K") derived from full length growth hormone (GH), the growth hormone variant (GH-V), placental lactogen (PL) or prolactin (PRL), preferably all of human origin. These 14-16 kD fragments having the activity of inhibiting plasminogen activator inhibitor 1 (PAl-1) are those having the sequences SEQ ID NOs: 2, 3, 4 (hPRL fragments), SEQ ID NO: 6 (hPL fragment), SEQ ID NO: 8 (hGH fragment) and SEQ ID NO: 10 (hGH-v fragment) shown in the sequence listing.

As defined, herein peptides "substantially identical" in amino acid sequence are also included in the scope in the invention. The about 14-16K N-terminal fragments of the full length hormones are also referred to herein as "16K N-terminal human growth hormone" (16K hGH), "16K N-terminal human placental lactogen" (16K hPL), "16K N-terminal growth hormone variant hGH-V" (16K hGH-V), and "16K N-terminal human prolactin" (16K hPRL).

### Production of the Peptides of the Invention

The peptides of the current invention can, for example, be synthesized, prepared from purified full-length hormones, or produced using recombinant methods and techniques known in the art. Although specific techniques for their preparation are described herein, it is to be understood that all appropriate techniques suitable for production of these peptides are intended to be within the scope of this invention.

Generally, these techniques include DNA and protein sequencing, cloning, expression and other recombinant engineering techniques permitting the construction of prokaryotic and eukaryotic vectors encoding and expressing each of the peptides of the invention.

In one mode, the peptides of this invention are conveniently obtained by isolation of intact growth hormone from the human pituitary gland or plasma and isolation of lactogen and growth hormone variant hGH-V. The isolated intact hormones may be glycosylated and cleaved to varying degrees.

In another mode, the peptides may be prepared by peptide synthesis according to method described in Biotechnology and Applied Biochem., 12:436 (1990) or by methods described in Current Protocols in Molecular Biology, Eds. Ausubel, F.M., et al, John Wiley & Sons, N.Y. (1987).

The peptides of the invention may be produced by expression of a nucleic acid encoding a protein or peptide of interest, or by cleavage from a longer length polypeptide encoded by the nucleic acid. Expression of the encoded polypeptides may be done in bacterial, yeast, plant, insect, or mammalian hosts by techniques well known in the art.

In an embodiment, a peptide of interest of the invention is obtained by cloning the DNA sequence encoding an intact full length human hormone into a vector; modifying the DNA codon corresponding to the last amino acid of a desired 16K N-terminal hormone fragment to a stop codon by mutagenesis techniques known in the art; and transforming a host cell with the modified nucleic acid to allow expression of the encoded peptide. In a further embodiment, the cloned hormone DNA is engineered to create a proteolytic cleavage site within the hormone polypeptide. The polypeptide is then cleaved after production in the host to generate the peptide of interest.

Examples of mutagenesis techniques include, for example, methods described in Promega Protocols and Applications Guide, Promega Corp, Madison, WI, p.98 (1891) or according to Current Protocols in Molecular Biology, supra.

If the peptide is to be synthesized in a prokaryotic vector, the DNA sequence encoding human hormone preferably does not contain a signal peptide sequence. In addition, a DNA codon for methionine (Met) is typically inserted upstream of 5' to the first DNA codon of the coding sequence.

Methods for cloning DNA into a vector and for inserting, deleting and modifying polynucleotides and for site directed mutagenesis are described, for example, in Promega Protocols and Applications Guide, supra. Cells or bacteria may be transfected with a vector, preferably with an expression vector, having the desired DNA sequence attached thereto, by known techniques including heat shock, electroporation, calcium phosphate precipitation and lipofection, among others. The terminal peptides or other analogues or fragments may then be extracted and purified by, for example, high pressure liquid chromatography (HPLC), ion exchange chromatography or gel permeation chromatography.

The following terms are used to describe the sequence relationships between two or more nucleic acids or polynucleotides: "reference sequence", "comparison window", "sequence identity", "percentage of sequence identity", and "substantial identity". A "reference sequence" is a defined sequence used as a basis for a sequence comparison; a reference sequence may be a subset of a larger sequence, for example, as a segment of a full-length cDNA or gene sequence given in a sequence listing, or may comprise a complete cDNA or gene sequence.

Optimal alignment of sequences for aligning a comparison window may, for example, be conducted by the local homology algorithm of Smith and Waterman Adv. Appi. Math. 2:482 (1981), by the homology alignment algorithm of Needleman and Wunsch J. Mol. Biol. 48:443 (1970), by the search for similarity method of Pearson and Lipman proc. Natl. Acad. Sci. U.S.A. 85:2444 (1988), or by computerized implementations of these algorithms (GAP, BESTFIT, FASTA, and TFASTA in the Wisconsin Genetics Software Package Release 7.0, Genetics Computer Group, 575 Science Dr., Madison, WI).

As applied to polypeptides, the terms "substantial identity" or "substantial sequence identity" mean that two peptide sequences, when optimally aligned, such as by the programs GAP or BESTFIT using default gap weights, share at least 80 percent sequence identity, preferably at least 90 percent sequence identity, more preferably at least 95 percent sequence identity or more. "Percentage amino acid identity" or "percentage amino acid sequence identity" refers to a comparison of the amino acids of two polypeptides which, when optimally aligned, have approximately the designated percentage of the same amino acids. For example, "95% amino acid identity" refers to a comparison of the amino acids of two polypeptides which when optimally aligned have 95% amino acid identity. Preferably, residue positions which are not identical differ by conservative amino acid substitutions.

For example, the substitution of amino acids having similar chemical properties such as charge or polarity are not likely to effect the properties of a protein. Examples include glutamine for asparagine or glutamic acid for aspartic acid.

"Homologous" amino acid residues as used herein refer to amino acid residues which have similar chemical properties concerning hydrophobicity, charge, polarity, steric features, aromatic feature etc. Examples for amino acids which are homologous to each other include in terms of positive charge lysine, arginine, histidine; in terms of negative charge: glutamic acid, aspartic acid; in terms of hydrophobicity: glycine, alanine, valine, leucine, isoleucine, proline, phenylalanine; in terms of polarity serine, threonine, cysteine, methionine, tryptophan, tyrosine, asparagine, glutamine; in terms of aromaticity: phenylalanine, tyrosine, tryptophan; in terms of chemically similar side groups: serine and threonine; or glutamine and asparagines; or leucine and isoleucine.

The phrase "substantially purified" or "isolated" when referring to a peptide or protein, means a chemical composition which is essentially free of other cellular components. It is preferably in a homogeneous state although it can be in either a dry or aqueous solution. Purity and homogeneity are typically determined using analytical chemistry techniques such as polyacrylamide gel electrophoresis or high performance liquid chromatography. A protein which is the predominant species present in a preparation is substantially purified. Generally, a substantially purified or isolated protein will comprise more than 80% of all macromolecular species present in the preparation. Preferably, the protein is purified to represent greater than 90% of all macromolecular species present. More preferably the protein is purified to greater than 95%, and most preferably the protein is purified to essential homogeneity, wherein other macromolecular species are not detected by conventional techniques.

### Nucleic Acids of the Invention

Also provided herein are isolated nucleic acids that comprise DNA or RNA sequences encoding the peptides of the invention. The nucleic acids of the invention may further comprise vectors for expression of the peptides of the invention. In some embodiments the DNA may comprise cDNA sequences encoding full-length hormones or sequences encoding N-terminal regions of the hormones. It is understood by one of ordinary skill in the art that because of degeneracy in the genetic code, substitutions in the nucleotide sequence may be made which do not result in changes in the encoded amino acid sequence. Thus, "substantially identical" sequences as defined herein are included in the scope of the invention. It is further understood by one of ordinary skill in the art that both complementary strands of any DNA molecule described herein are included within the scope of the invention.

The terms "substantial identity" or "substantial sequence identity" as applied to nucleic acid sequences and as used herein and denote a characteristic of a polynucleotide sequence, wherein the polynucleotide comprises a sequence that has at least 85 percent sequence identity, preferably at least 90 to 95 percent sequence identity, and more preferably at least 99 percent sequence identity as compared to a reference sequence over a comparison window of at least 20 nucleotide positions, frequently over a window of at least 25-50 nucleotides, wherein the percentage of sequence identity is calculated by comparing the reference sequence to the polynucleotide sequence which may include deletions or additions which total 20 percent or less of the reference sequence over the window of comparison. The reference sequence may be a subset of a larger sequence.

### Treatment Protocols

The method for treatment of thrombosis-related diseases or conditions relating to high level of PAI-1 expression comprises administering to a patient an PAI-1 inhibitory amount of one or more peptides of the invention.

As used herein, the term "treatment" is intended to refer to the prevention, amelioration, or reduction in severity of a symptom of thrombosis-related diseases or conditions relating to high level of PAI-1 expression. Similarly, an effective dose of a protein or peptide according to the invention is a dose sufficient to prevent, ameliorate, or reduce the severity of a symptom of thrombosis-related diseases or conditions relating to high level of PAI-1 expression.

The peptides of the invention may be administered singly or in combination with each other or other fibrinolytics.

Typically, the proteins or peptides are administered in an amount of about 8 micrograms to 3,000 µg/kg per day, and more preferably about 20 to 1,500 µg/kg per day preferably once or twice daily. However, other amounts, including substantially lower or higher amounts, may also be administered. The peptides of the invention are administered to a human subject in need of treatment intramuscularly, subcutaneously, intravenously, by any other acceptable route of administration.

For this or any other this application the peptide of this invention may be administered in an amount of about 10 to 3,750 µg/kg, and more preferably about 15 to 1,600 µg/kg. Any means of administration is suitable.

### Gene Therapy

Gene therapy utilizing recombinant DNA technology to deliver nucleic acids encoding proteins and peptides of the invention into patient cells or vectors which will supply the patient with gene product in vivo is also contemplated within the scope of the present invention.

Gene therapy techniques have the potential for limiting the exposure of a subject to a gene product, such as polypeptide, by targeting the expression of the therapeutic gene to a tissue of interest, such as skeletal muscle, myocardium, vascular endothelium or smooth muscle, or solid or circulating tumor cells. For example, WIPO Patent Application Publication No. WO 93/15609 discloses the delivery of interferon genes to vascular tissue by administration of such genes to areas of vessel wall injury using a catheter system.

In another example, an adenoviral vector encoding a protein capable of enzymatically converting a prodrug, a "suicide gene", and a gene encoding a cytokine are administered directly into a solid tumor.

Other methods of targeting therapeutic genes to tissues of interest include the three general categories of transductional targeting, positional targeting, and transcriptional targeting. Transductional targeting refers to the selective entry into specific cells, achieved primarily by selection of a receptor ligand. Positional targeting within the genome refers to integration into desirable loci, such as active regions of chromatin, or through homologous recombination with an endogenous nucleotide sequence such as a target gene. Transcriptional targeting refers to selective expression attained by the incorporation of transcriptional promoters with highly specific regulation of gene expression tailored to the cells of interest.

Elements aiding specificity of expression in a tissue of interest can include secretion leader sequences, enhancers, nuclear localization signals, endosmolytic peptides, etc. Preferably, these elements are derived from the tissue of interest to aid specificity.

Viral vector systems useful in the practice of the instant invention include but are not limited to adenovirus, herpesvirus, adeno-associated virus, minute virus of mice (MVM), HIV, sindbis virus, and retroviruses such as Rous sarcoma virus, and MoMLV. Typically, the nucleic acid encoding the therapeutic polypeptide or peptide of interest is inserted into such vectors to allow packaging of the nucleic acid, typically with accompanying viral DNA, infection of a sensitive host cell, and expression of the polypeptide or peptide of interest.

The nucleic acid can be introduced into the tissue of interest in vivo or ex vivo by a variety of methods. In some embodiments of the invention, the nucleic acid is introduced to cells by such methods as microinjection, calcium phosphate precipitation, liposome fusion, or biolistics. In further embodiments, the nucleic acid is taken up directly by the tissue of interest. In other embodiments, nucleic acid is packaged into a viral vector system to facilitate introduction into cells.

In some embodiments of the invention, the compositions of the invention are administered ex vivo to cells or tissues explanted from a patient, then returned to the patient.

### Formulations and Pharmaceutical Compositions

The compositions of the invention will be formulated for administration by manners known in the art acceptable for administration to a mammalian subject, preferably a human. In some embodiments of the invention, the compositions of the invention can be administered directly into a tissue by injection or into a blood vessel supplying the tissue of interest. In further embodiments of the invention the compositions of the invention are administered "locoregionally", i.e., intravesically, intralesionally, and/or topically. In other embodiments of the invention, the compositions of the invention are administered systemically by injection, inhalation, suppository, transdermal delivery, etc.

In further embodiments of the invention, the compositions are administered through catheters or other devices to allow access to a remote tissue of interest, such as an internal organ. The compositions of the invention can also be administered in depot type devices, implants, or encapsulated formulations to allow slow or sustained release of the compositions.

In order to administer therapeutic agents based on, or derived from, the present invention, it will be appreciated that suitable carriers, excipients, and other agents may be incorporated into the formulations to provide improved transfer, delivery, tolerance, and the like.

A multitude of appropriate formulations can be found in the formulary known to all pharmaceutical chemists: Remington's Pharmaceutical Sciences, (15th Edition, Mack Publishing Company, Easton, Pennsylvania (1975)), particularly Chapter 87, by Blaug, Seymour, therein. These formulations include for example, powders, pastes, ointments, jelly, waxes, oils, lipids, anhydrous absorption bases, oil-in-water or water-in-oil emulsions, emulsions carbowax (polyethylene glycols of a variety of molecular weights), semi-solid gels, and semi-solid mixtures containing carbowax.

Any of the foregoing formulations may be appropriate in treatments and therapies in accordance with the present invention, provided that the active agent in the formulation is not inactivated by the formulation and the formulation is physiologically compatible.

The quantities of active ingredient necessary for effective therapy will depend on many different factors, including means of administration, target site, physiological state of the patient, and other medicaments administered.

Thus, treatment dosages should be titrated to optimize safety and efficacy. Typically, dosages used in vitro may provide useful guidance in the amounts useful for in situ administration of the active ingredients. Animal testing of effective doses for treatment of particular disorders will provide further predictive indication of human dosage.

Various considerations are described, for example, in Goodman and Gilman's the Pharmacological Basis of Therapeutics, 7th Edition (1985), MacMillan Publishing Company, New York, and Reminaton's Pharmaceutical Sciences 18th Edition, (1990) Mack Publishing Co, Easton Penn. Methods for administration are discussed therein, including oral, intravenous, intraperitoneal, intramuscular, transdermal, nasal, iontophoretic administration, and the like.

The compositions of the invention may be administered in a variety of unit dosage forms depending on the method of administration. For example, unit dosage forms suitable for oral administration include solid dosage forms such as powder, tablets, pills, capsules, and dragees, and liquid dosage forms, such as elixirs, syrups, and suspensions.

The active ingredients may also be administered parenterally in sterile liquid dosage forms. Gelatin capsules contain the active ingredient and as inactive ingredients powdered carriers, such as glucose, lactose, sucrose, mannitol, starch, cellulose or cellulose derivatives, magnesium stearate, stearic acid, sodium saccharin, talcum, magnesium carbonate and the like. Examples of additional inactive ingredients that may be added to provide desirable color, taste, stability, buffering capacity, dispersion or other known desirable features are red iron oxide, silica gel, sodium lauryl sulfate, titanium dioxide, edible white ink and the like.

Similar diluents can be used to make compressed tablets. Both tablets and capsules can be manufactured as sustained release products to provide for continuous release of medication over a period of hours. Compressed tablets can be sugar coated or film coated to mask any unpleasant taste and protect the tablet from the atmosphere, or enteric-coated for selective disintegration in the gastrointestinal tract. Liquid dosage forms for oral administration can contain coloring and flavoring to increase patient acceptance.

The concentration of the compositions of the invention in the pharmaceutical formulations can vary widely, i.e., from less than about 0.1 %, usually at or at least about 2% to as much as 20% to 50% or more by weight, and will be selected primarily by fluid volumes, viscosities, etc., in accordance with the particular mode of administration selected.

The compositions of the invention may also be administered via liposomes. Liposomes include emulsions, foams, micelles, insoluble monolayers, liquid crystals, phospholipid dispersions, lamellar layers and the like. In these preparations the composition of the invention to be delivered is incorporated as part of a liposome, alone or in conjunction with a molecule which binds to a desired target, such as antibody, or with other therapeutic or immunogenic compositions. Thus, liposomes either filled or decorated with a desired composition of the invention can delivered systemically, or can be directed to a tissue of interest, where the liposomes then deliver the selected therapeutic/immunogenic peptide compositions.

Liposomes for use in the invention are formed from standard vesicle-forming lipids, which generally include neutral and negatively charged phospholipids and a sterol, such as cholesterol. The selection of lipids is generally guided by consideration of, e.g., liposome size, acid lability and stability of the liposomes in the blood stream. A variety of methods are available for preparing liposomes, as described in, e.g., Szoka et al. Ann. Rev. Biophys. Bioena. 9:467 (1980), U.S. Patent Nos. 4,235,871, 4,501,728, 4,837,028, and 5,019,369, incorporated herein by reference.

A liposome suspension containing a composition of the invention may be administered intravenously, locally, topically, etc. in a dose which varies according to, inter alia, the manner of administration, the composition of the invention being delivered, and the stage of the disease being treated.

For solid compositions, conventional nontoxic solid carriers may be used which include, for example, pharmaceutical grades of mannitol, lactose, starch, magnesium stearate, sodium saccharin, talcum, cellulose, glucose, sucrose, magnesium carbonate, and the like. For oral administration, a pharmaceutically acceptable nontoxic composition is formed by incorporating any of the normally employed excipients, such as those carriers previously listed, and generally 10-95% of active ingredient, that is, one or more compositions of the invention, and more preferably at a concentration of 25%-75%.

For aerosol administration, the compositions of the invention are preferably supplied in finely divided form along with a surfactant and propellant. Typical percentages of compositions of the invention are 0.01%-20% by weight, preferably 1%-10%. The surfactant must, of course, be nontoxic, and preferably soluble in the propellant.

Representative of such agents are the esters or partial esters of fatty acids containing from 6 to 22 carbon atoms, such as caproic, octanoic, lauric, palmitic, stearic, linoleic, linolenic, olesteric and oleic acids with an aliphatic polyhydric alcohol or its cyclic anhydride. Mixed esters, such as mixed or natural glycerides may be employed. The surfactant may constitute 0.1%-20% by weight of the composition, preferably 0.25-5%. The balance of the composition is ordinarily propellant. A carrier can also be included, as desired, as with, e.g., lecithin for intranasal delivery.

The compositions of the invention can additionally be delivered in a depot-type system, an encapsulated form, or an implant by techniques well-known in the art. Similarly, the compositions can be delivered via a pump to a tissue of interest.

The compositions of the invention are typically administered to patients after the onset of symptoms, although treatment can also be prophylactic in some embodiments.

Typically, treatment with direct administration of polypeptides is done daily, weekly, or monthly, for a period of time sufficient to reduce, prevent, or ameliorate symptoms.

The composition of the invention may also be provided in the kit as a slow-release composition such as a daily, weekly, monthly unit provided as a sponge, dermal patch, subcutaneous implant and the like in a wrapping or container as described above. In this case, the patient may release a unit of the composition from the container and applies it as indicated in the kit instructions. The composition may then be replaced at the end of the specified period by a fresh unit, and so on. The present composition may also be administered by means of injection, as indicated above. Typically, the peptide may be administered by itself. Similarly, the peptide of the invention may be administered in a composition that also comprises another drug. The proportion of peptides to the other drug(s) and carrier may be adjusted accordingly.

The levels of the delivered peptide to a patient may be monitored by immunoassay. To determine the level of the peptide of invention in blood following administration, e.g., intramuscular or subcutaneous administration, an antibody assay may be performed with antibodies specific to the peptide sequence by any of the protocols known in the art. Polyclonal or monoclonal antibodies may be utilized. The level of the peptide in blood may then be correlated with the progress of the inhibition of any of the diseases the patient is afflicted with.

### Examples:

The materials and methods used in the studies of the present invention (see examples 1 to 10) are presented below:

### Materials and methods:

Recombinant 16K hPRL was produced and purified from *E. coli* as previously described (WO 98/51323). Recombinant 14K hGH was produced and purified from *E. coli* as previously described (WO 98/51323). The purity of the recombinant protein exceeded 95% (as estimated by Coomassie blue staining) and the endotoxin level was respectively 0.5 pg/ng and 0.15 pg/ng of recombinant 16K hPRL and 14K hGH, as quantified with the « Rapid Endo Test » of the European Endotoxin Testing Service (Cambrex Bioscience, Verviers, Belgium). Recombinant wt PAI-1 were a gift of Pr. P.Declerck. uPA and tPA were purchased from Calbiochem.

### Cell Cultures

All cells were cultured at 37°C in a 5% CO₂ humid atmosphere except ABAE that were grown at 38°C. ABAE cells were isolated as previously described. The cells were grown and serially passaged in low-glucose DMEM containing 10% foetal bovine serum (FBS) and 100 U/ml penicillin/streptomycin. Recombinant bFGF (Sigma) was added (1 ng/ml) to the culture every other day. Confluent cells corresponding to passages 8 to 13 were used in the experiment. HUVEC cultures were isolated as previously described and maintained in EBM2 medium (Cambrex Bio Science Walkersville, Walkersville, USA) containing 0.1% hEGF, 0.04% hydrocortisone (kit EGM-2 SingleQuots, Cambrex Bio Science Walkersville, Walkersville, USA), 10% FBS, and 100 U/ml penicillin/streptomycin. For assays, serum-free medium was also used (Invitrogen). B16-F10 mouse melanoma cells were obtained from the American Type Culture Collection (ATCC CRL-6475, Rockville, Md) and cultured in high-glucose DMEM supplemented with 10% FCS and 4 mM glutamine, 100 units/ml penicillin, 100 µg/ml streptomycin.

### Statistical analysis

All data were expressed as means ± SD except when indicated. Data for *in vitro* proliferation, apoptosis assays. Tumors growth curves data were analysed using the Mann-Whitney U-test performed with Prism 4.0 software (GraphPad Software, San Diego, CA, USA). Statistical significance was set at P < 0.05.

### Example 1: Detecting the interaction partner of 16K hPRL by the Yeast-Two Hybrid system

In a search for potential 16K PRL targets, the Gal4-based yeast two-hybrid system was used to screen a HUVEC cDNA library for sequences encoding proteins interacting with 16K hPRL. For this, the 16K hPRL cDNA was cloned in frame with the Gal4 DNA-binding domain of the yeast two-hybrid vector.

Yeast two-hybrid screening was performed using the MATCHMAKER GAL4 Two-Hybrid System 3 (Clontech) according to the manufacturers instructions. 16K hPRL cDNA (stop140) was used as a bait and cloned into pGBKT7 vector in frame with the GAL4 DNA-binding domain (pBD-16K). The construct was tested for absence of transcriptional activation and toxicity. Subsequently, yeast *AH109* cells were co-transformed with pBD-16K, Smal-linearized prey vector (pGADT7), and a cDNA library which was generated from activated HUVEC mRNA. Following growth on media plates selective for reporter gene activation, prey plasmids from positive yeast colonies were isolated using CHROMA SPIN-1000 columns (Clontech), shuttled into *Escherichia coli,* and sequenced at the genomic plateform of the GIGA-Research center. Confirmation of interaction was performed by targeted transformation of the specific constructs using the small-scale yeast transformation protocol as described in the yeast protocol handbook (Clontech). Those specific constructs were: 14K hGH cDNA cloned in pGKBT7, full-length human PAI-1 cloned in pGADT7, pBD16K or cloned isolated from the screen.

Among the 20 clones isolated in this screen, four clones encoded parts of the PAI-1 sequence. Alignment of the clones (Fig.1a) identified two regions in common between at least 3 different clones. Interestingly, the locations of these regions on the PAI-1 3D-structure revealed their close proximity to the RCL, the reactive-center loop of active PAI-1 (Fig.1b). The full-length PAI-1, cloned into the Gal4-prey vector, reconstituted the two-hybrid interaction in yeast. In addition the 14K hGH, cloned into the Gal4-bait vector, was also found to interact with the PAI-1.

### Example 2: Detecting the interaction of 16K hPRL and 14K hGH with PAI-1 by Surface Plasmon Resonance

Real-time monitoring of molecular interactions was performed at 25°C using the BIAcore 1000 biosensor system (BIAcore) according to the manufacturer's instructions. Recombinant PAI-1 was immobilized to a CM5 sensor chip (BIAcore) via primary amine groups using the Amine Coupling Kit (BIAcore). For interaction analysis, 20 µl sample was diluted to various concentrations in HBS-EP (BIAcore) and was injected using the KINJECT command at a flow rate of 30 µl/minute after which the flow cells were regenerated by injection of 20 µl of regeneration buffer (10 mM glycine-HCI, pH 2.0). Association-rate (ka) and dissociation-rate (kd) constants were obtained by analysis of the sensorgrams using the Biaevaluation software, version 3.2. All measurements were performed at least in duplicate at all concentrations and the experiment was performed repeated 3 times.

The surface plasmon resonance (Biacore analysis) confirmed the interaction of 16K hPRL with PAI-1. The analysis of the binding kinetics revealed that 16K hPRL and 14K hGH bind to PAI-1 with strong affinity, the K_{d} being 6x10⁻⁷ M for 16K hPRL and 1 x10⁻⁶ M for 14K hGH (Fig 1 c).

### Example 3: Detecting the interaction of 16K hPRL and 14K hGH with PAI-1 by immunoprecipitation

The interaction was further confirmed by pull-down affinity assays. For the immunoprecipitation 4µg of recombinant 16K hPRL or 14 hGH were mixed with 3µg of recombinant wt PAI-1, 500µl of conditioned media from HUVEC cells, 300µl of human plasma or 100µl of mouse plasma. The mixtures were incubated for 10min at 37°c. 6µg of mouse monoclonal antibody against PAI-1 (clone 33H1F7, recognize human and mouse PAI-1) was added to each tube and incubated overnight at 4°C under rotation. For the experiments with mice expressing 16K hPRL, 300µl of plasma from mice injected with Null-Ad or 16K-Ad adenovirus were collected 5 days after virus injection, and immunoprecipitation was performed as described above with same PAI-1 monoclonal antibody (33H1 F7). Fifty microliters of protein A agarose was added to the mix and incubated overnight at 4°C under rotation. After centrifugation for 1 min at 6000 rpm the supernatants were discarded and the pellets (containing the beads) were washed 5 times with PBS. After a new centrifugation, the beads were resuspended in 30 µl 1X β-mercaptoethanol loading buffer and boiled at 100°C for 5 min, then left on ice for 5 min. The beads were centrifuged for 5 min at 4°C, then the supernatants were subjected to SDS-PAGE (12% acrylamide) for Western blot analysis.

For Western Blot Analysis cytoplasmic cell lysate, samples of different CoIP and mouse plasma were separated by SDS-PAGE (12% and 10%) and transferred to a polyvinylidene fluoride membrane (Milipore Corp., Bedford, MA). Blots were blocked overnight with 5% milk in Tris-buffered saline with 0.1% Tween 20 and probed for 1 h at RT with primary antibodies: rabbit polyclonal anti-hPRL (DAKO) or rabbit polyclonal anti hGH (home made). After three washes with Tris-buffered saline containing 0.1 % Tween 20, antigen-antibody complexes were detected with peroxidase-conjugated goat anti-rabbit secondary antibody and an enhanced fluoro-chemiluminescent system (ECL-plus; Amersham Biosciences, Arlington Heights, IL).

The immunoprecipitation revealed that the incubation for 10 min at 37°C of recombinant 16K PRL or 14K GH with recombinant wild type PAI-1, lead to the formation of 16K PRL/PAI-1 or 14K GH/PAI-1 (Fig. 2a). The recombinant 16K PRL and 14K GH were also able to form a complex with endogenous PAI-1 produced in culture medium by human umbilical vein endothelial cells (HUVEC) (Fig. 2b). Furthermore, the interaction was also demonstrated between 16K PRL or 14K GH and PAI-1 from either human or mouse origin in plasma samples (Fig. 2c).

### Example 4: Detecting the inhibition of PAI-1 anti-proteolytic activity

The main physiological function of PAI-1 is to inhibit uPA and tPA proteolytic activity. The inhibition of uPA proteolytic activity was tested as follows: uPA was incubated with bovine plasminogen (Sigma, 0,1 mg/ml) in the presence of PAI-1 and their potential inhibitors such as 16K hPRL and 14K hGH. A chromogenic substrate for plasmin (Spectrozyme PL: 0,5mM. American Diagnostica INC) in PBS was added to the mixture in 96-well microtitration plates and kept at room temperature. The release of free p-nitro-aniline from the chromogenic substrate was measured spectrophotometrically. The activity of plasmin was determined as the absorbance at 405nm.

As result it could be shown that the physiological function of PAI-1 to inhibit uPA and tPA proteolytic activity function was significantly inhibited by 16K PRL and 14K GH. However, the full fragment 23K PRL or 22K GH did not show any inhibition of PAI-1 anti-proteolytic activity (Fig 3a).

### Example 5: Zymographic analysis

Zymographic assays showed that the inhibition of plasmin (Pln) generation in the presence of PAI-1 was inhibited by the addition of 14K PRL whereas the full GH has no effect (Fig. 3b). For this test casein (1mg/ml) was incorporated in 10% SDS polyacrylamide gels. Aliquots of *in vitro* reaction including recombinant proteins were mixed with loading buffer (10%SDS, 4% Sucrose, 0.25M Tris-HcI pH6-8, 0.1% Bromophenol blue). After electrophoresis, SDS was removed from the gel by two washes (30min each) of 100mM glycine, 2.5% Triton buffer (pH 8.0) stained with 0.1% coomassie brilliant bleu, and destained in 20% methanol/10% acetic acid.

### Example 6: Analysis of complex formation between PAI-1 and tPA by SDS-PAGE

tPA/PAI-1 complex was formed 10min after their incubation at 37°C. Preincubation of PAI-1 (4µg) with 16K hPRL (5µg) or 14K hGH (4µg) during 10min at 37°C, followed by a second 10min incubation with tPA (2µg) was stopped by the addition of sample buffer containing 5% β-Mercaptoethanol. Samples are heated at 95°C before being analysed by SDS-PAGE. Silver staining was performed on the gel to visualize protein bands and complexes.

The results showed that 16K PRL was able to partially inhibit PAI-1/tPA complex formation, and the 14K GH completely abolished PAI-1/tPA complex formation (Fig. 3c). Similar results were obtained when using uPA instead of tPA (data not shown).

### Example 7: Detecting the inhibition of PAI-1 anti-proteolytic activity by in vitro clot lysis assays

Since PAI-1 also plays important role in thrombosis, the effects of 16K fragments and specific small peptides of said 16 K fragments on clot dissolution were further analysed.

Clot lysis was performed as follows: pooled normal plasma were used in citrated tube. Recombinant wild type PAI-1 was incubated in the presence or in the absence of 16K hPRL (3µM), 14K hGH (3µM) or different peptides (25 µM) for 10min at 37°C, before to be added to diluted plasma in tris tween buffer (75µl of plasma in 200µl of 10mM TRIS pH 7.5, 0.01% tween 20), followed by a second incubation for 10min at 37°C. tPA was added to each tube at the end to reach a final volume of 200µl. Aliquots from each tube (80µl) were added in duplicate to 96 wells, each containing 20µl CaCl₂ to give the following final concentration per well of 10 mM. Clot formation and dissolution were monitored at RT measuring turbidity at 405nm in 5 min intervals.

As result, it was found that the inhibition of PAI-1 by 16K PRL or 14K GH increased significantly the clot lysis in human plasmas as shown by *in vitro* clot lysis assays (Fig. 4a).

Furthermore designed shorter peptides either from 16K PRL or 14K GH (see Fig. 4c.) were able to inhibit PAI-1 anti-proteolytic activity in a colorimetric test (data not shown), and to significantly increase the clot lysis in the presence of endogenous added PAI-1. For example, one 6-aminoacid-peptide derived from 14K GH: POGH 5-10 turnded out to be as effective as the tilted peptide of GH: POGH (Fig. 4b). Table 1 summarizes the results of the peptide fragments of X1-X14.

**Table 1: Peptide fragments inhibiting PAI-1.**

| **name of the peptide*** | **sequence** | **PAI-1 activity in vitro (chromogenic assay)** | **ablility to induce clot lysis** | **SEQ ID NO:** |
|---|---|---|---|---|
| POGH | LLRISLLLIQSWLE | yes | yes | 12 |
| POGH 1-6 | LLRISL | yes | yes | 15 |
| POGH 3-8 | RISLLL | yes | yes | 16 |
| POGH 5-10 | SLLLIQ | yes | yes | 17 |
| POGH 9-14 | IQSWLE | yes | yes | 19 |
| POGHmut** | LSQILSSLIQSWLE | no | no | 32 |
| POPRL 1-6 | FLSLIV | yes | yes | 23 |
| POPRL 5-10 | IVSILR | yes | yes | 25 |

| | | | | |
|---|---|---|---|---|
| *) PO = peptide oblique = tilted peptide; GH = growth hormone; hPRL = prolactin; mut= mutated **) negative control | | | | |

### Example 8: In vitro proliferation, migration and adhesion assays with endothelial cells in the presence or in the absence of PAI-1

In order to investigate if PAI-1/16K PRL and PAI-1/14K GH interactions could be responsible for known-functions of 16K PRL and 14K GH, *in vitro* proliferation, migration and adhesion assays were carried on with endothelial cells in the presence or in the absence of PAI-1 (Fig. 5a,b,c).

### Analysis of cell proliferation

The cells were plated in 96-well culture plates at a density of 2,500 cells per well in 10% FCS/DMEM and allowed to adhere for 24 h. Then complete medium was replaced with SFM for 24 h. When indicated the cells were treated by bFGF (10ng/ml) with or without 16K hPRL (5nM), 14K hGH (5nM) and PAI-1 (20nM) for 15 min at room temperature. Proliferation was analysed 24 h later by measuring BrdU incorporation by means of the Cell Proliferation ELISA, BrdU (Colorimetric) (Roche, Clinical Laboratories, Indianapolis, IN).

### Adhesion assay

96 well, non-tissue culture-treated plates were coated with 100µl/well of native vitronectin (10 µg/ml, Millipore) at room temperature for 2 h. Non specific binding was blocked by the addition of 1% heat-treated BSA for 30 min at room temperature. Non confluent cells are treated with trypsin, followed by neutralization with serum-containing medium, were washed with PBS (137mM NaCl, 2.6mM KCI, 10mM Na₂HPO₄ and 1.7mM KH₂PO₄, pH 7.4) and resuspended in 100µl of adhesion buffer (serum free medium containing 0,5% BSA, 1mM CaCl₂, 1mM MgCl₂ and 0.2mM MnCl₂). When indicated, 16K hPRL and/or 14K hGH (50nM) were incubated with recombinant hPAI-1 (100nM) for 15min at room temperature prior to the addition of cells. The cells were plated in the wells (50000 cells/well) and allowed to adhere for 1 h. Cells were then washed three times with adhesion buffer to remove non-adherent cell. The adherent cells were stained with a 0.1% crystal violet in methanol at room temperature for 5min. After three washing, the insoluble dye taken up by adherent cells was dissolved in 200µl of methanol. Quantification was performed by reading the optical density at 570 nm with a microplate reader (Wallac Victor²; Perkin Elmer, Norwalk, Finland).

### siRNA Transfections

Small interfering RNA (siRNA) duplexes were obtained from Integrated DNA Technologies (Integrated DNA Technologies, Coralville, USA), two targeting bovine PAI-1 and one negative control (Ambion). Cells were transfected (110,000 cells per well in 12-well plate for adhesion assay and 11,000 cells per well in 96-well plate for proliferation assay) by using Siport neofx tranfection reagent according to the manufacturer's protocol. Briefly, ABAE cells were transfected with a siRNA PAI-1 or a control siRNA (20nM) in a medium SFM containing 5ng/ml bFGF and 0.5ng/ml EGF. For *in vitro* assays, cells were used 96 hours after transfection. Sequence for PAI-1 siRNA dicer substrate (IDT) are PAI-1 si-RNA 1 5'-CCG AUUUACUGAAGAAUUGCACAGA-3' (SEQ ID NO: 35); PAI-1 si-RNA 2: 5'-GAAUGUAACCUAAUAGAACCCUAAU-3' (SEQ ID NO: 36). All transfection experiments shown in this application have been made with PAI-1 si1. Similar results were obtained with PAI-1 si2 (data not shown).

As result, it was shown that the addition of recombinant wild type PAI-1 to culture medium reduced significantly the anti-proliferation, the pro-adhesion and the anti-migratory effects of 16K PRL and 14K GH (Fig. 5d,e,f). This suggested that the excess of exogenous added recombinant PAI-1 in the medium could titrate 16K PRL or the 14K GH in the medium, rendering it inaccessible to the cells. Conversely, the down-regulation of PAI-1 expression using si-RNA interference method, in endothelial cells, reduced significantly the anti-proliferative and the anti-migratory function of 16K PRL and 14K GH. The increase of cell adhesion by 16K PRL or 14K GH was not seen in the absence of PAI-1. In order to confirm that PAI-1 was required for the anti-angiogenic effect of 16K PRL, recombinant PAI-1 was added in cells transfected with PAI-1 si-RNA. Indeed, the addition of human PAI-1 restored the anti-migratory effect of 16K PRL and 14K GH (Fig. 5g).

### Example 9: PAI-1 is required for antitumoral activity of 16K PRL

Next it was analysed whether PAI-1 expression is required for anti-tumoral effect of 16K PRL.

### Mice

Homozygous PAI-1 deficient mice (PAI-1 ^{-/-}) and the corresponding WT mice (PAI ^{+/+}) from either sex with a mixed genetic background 87% C57BL/6 and 13% 129 strain were used throughout this study. The animal experiment protocol used was approved by the Institutional Ethics Committee of the University of Liège.

### Mouse xenograft tumor model

Subconfluent B16-F10 cells were trypsinized, washed, and resuspended in PBS. Cell suspension (10⁵ cells in 50µl PBS) was injected subcutaneously into the right flank of mice experiments were performed on seven mice in each group. 18 to 20 days after tumor cells injection, the mice were killed and their tumors harvested. Tumor growth was assessed by measuring the length and width of each tumor every 1 to 2 days and calculating tumor volume by means of the formula: length x width² x 0.5.

### Adenovirus vectors

16K-Ad is a defective recombinant E1-E3-deleted adenovirus vector encoding a secreted polypeptide consisting of the first 139 amino acids of PRL. This adenovirus vector was constructed as previously described with the help of the Adeno-X expression system (BD Biosciences, Erembodegem, Belgium). Null-Ad is a control adenovirus carrying an empty expression cassette. A recombinant adenovirus vector bearing WT human PAI-1 (AdCMVPAI-1) and control adenovirus (AddRR5) were generated as previously described.

The 16K hPRL adenovirus and their corresponding control were injected into mouse tail vein 2 days before the subcutaneous injection of tumor cells. A second injection was done 8 days after the first one. The wt PAI-1 adenovirus and their control were injected one day after the 16K hPRL adenovirus injections. 10⁹ pfu of adenoviruses were used for each tail vein injection.

### ELISA for PAI-1 detection

PAI-1 level in mouse plasma was determined using commercially available ELISA kit for human PAI-1 (American Diagnostica Inc).

As previously described, the subcutaneous injection of B16F10 into syngenic mice, followed by a systemic expression of 16K PRL using adenoviral vector (16K-Ad), showed a significant reduction of tumor growth compared to mice injected with control adenovirus (Null-Ad).

However, 16K PRL expression did not show a tumor growth reduction in PAI-1 deficient mice (Fig. 6a). The level of 16K PRL expression was similar in wild type-or in the PAI-1 deficient-mice (Fig. 6b). Immunoprecipitation of PAI-1 in plasma of wild type mice, 5 days after 16K Ad or Null-Ad injection and immuno-blotting of 16K PRL, showed a complex formation between endogenous murine PAI-1 and 16K PRL expressed in mice circulation. In order to confirm that PAI-1 is required to induce the anti-tumoral effect of 16K PRL, PAI-1 expression was restored in PAI-1 deficient mice using adenoviral vector allowing hPAI-1 expression. Indeed, the systemic expression of human PAI-1 in PAI-1 deficient mice, restored the anti-tumoral effect of 16K PRL (Fig. 7b). The average plasma level of human wild type PAI-1 in seven mice was 2.83 ± 0.48 µg/ml, and no human PAI-1 was detected in PAI-1 deficient mice injected with control adenovirus (Fig. 7a). Interestingly, 5 days after adenovirus injection, the plasma level of 16K PRL is similar in PAI-1 deficient mice and the mice where PAI-1 expression was restored. However, at the end of the experiment, albeit the 16K PRL remained detectable in plasma of PAI-1 deficient mice, it considerably dropped off in plasma of mice where human PAI-1 expression was restored. Those *in vivo* data suggested that PAI-1 was required for antitumoral activity of 16K PRL and may act as a molecule that clears 16K PRL from plasma.

### Example 10: Immunocytochemical localization of uPA and 16K hPRL/14K hGH.

HUVEC were grown to 70% on gelatin-coated 35 mm dishes. After incubation at 37°C with 14K hGH (160ng/ml) or 16K hPRL (160ng/ml), cells were washed with PBS at room temperature, non permeabilized and incubated for 1 hr at room temperature with mouse anti uPA (American Diagnostica INC. Greenwich,CT) 1/100, Rabbit anti Prolactin (Dako) 1/100 or rabbit anti human GH (home made) 1/100. Endothelial cells were washed in PBS and incubated with FITC horse anti mouse antibody (Vector Laboratory) and Texas Red Goat anti rabbit (Jackson Laboratory).

So far, no receptor for PAI-1 has been described. Nevertheless, PAI-1 is internalized inside the cell when it forms a triplicate complex with uPA and uPAR on cell surface. This internalization involved an endocytotic receptor-related protein (LRP). Immunoprecipitation assays showed that 16K PRL or 14K GH does not form complex with uPA (data not shown). However, in the presence of PAI-1, immunoprecipitation assays revealed formation of uPA/PAI-1/16K PRL or uPA/PAI-1/14K GH complexes (Fig. 8a). Double immunofluorescence staining for uPA and 16K PRL or 14K GH, showed that uPA and 16K PRL or uPA and 14K GH co-localised at the cell surface (Fig. 8b). The results suggest that 16K PRL or 14K GH might exert their effects at the cell surface where it can bind uPA and PAI-1 complex.

### Conclusion

In conclusion, the data presented in this application showed that PAI-1 mediates 16K PRL and 14K GH activities. In addition, the data obtained during the studies according to the present invention provide evidence that 16K PRL, 14K GH or isolated peptides thereof blocked the ability of PAI-1 to inhibit plasminogen activators activities. Importantly, both 16K PRL, 14K GH or isolated peptides thereof induced clot lysis *in vitro.* These data allows to propose the use of the fragments, alone or in combination with fibrinolytics to dissolve clots for the treatment of thrombotic disorders like acute myocardial infraction or stroke.

## Claims

1. A pharmaceutical composition for use in the preventive and/or therapeutic treatment of thrombosis-related diseases or conditions relating to high level of PAI-1 expression, which composition is comprising
A) a fragment of a polypeptide or protein of prolactin (PRL)-growth hormone (GH) - placental lactogen (PL)-family and homologous derivatives thereof, which fragment comprises from 6 to 14 consecutive amino acid residues of the amino acid sequence having the following general formula (I):
X1-X2-X3-X4-X5-X6-X7-X8-X9-X10-X11-X12-X13-X14, wherein
X1 is an amino acid residue of: Leu, Phe;
X2 is an amino acid residue of: Leu;
X3 is an amino acid residue of: Arg, Ser;
X4 is an amino acid residue of: Ile, Leu;
X5 is an amino acid residue of: Ser, Ile;
X6 is an amino acid residue of: Leu, Val;
X7 is an amino acid residue of: Leu, Ser,
X8 is an amino acid residue of: Leu, Ile;
X9 is an amino acid residue of: Ile, Leu;
X10 is an amino acid residue of: Gln, Glu, Arg;
X11 is an amino acid residue of: Ser;
X12 is an amino acid residue of: Trp;
X13 is an amino acid residue of: Leu, Asn;
X14 is an amino acid residue of: Glu, (SEQ ID NO: 11); or
B) a peptide or a recombinant protein comprising said peptide, wherein the peptide is from 6 to 50 amino acids in length and has the activity to inhibit plasminogen activator inhibitor I (PAI-1), said peptide comprising from 6 to 14 consecutive amino acid residues of the amino acid sequence of said general formula (I); or
C) a polynucleotide encoding said fragment of a polypeptide or protein of A) or said peptide or recombinant protein of B).

2. The pharmaceutical composition according to claim 1, wherein the amino acid sequence X1-X14 is having at least 71%, preferably at least 78%, more preferably at least 85% and most preferred at least 92% identity to one of the following sequences:
a) Leu Leu Arg Ile Ser Leu Leu Leu Ile Gln Ser Trp Leu Glu (SEQ ID NO: 12);
b) Leu Leu Arg Ile Ser Leu Leu Leu Ile Glu Ser Trp Leu Glu (SEQ ID NO: 13);
c) Phe Leu Ser Leu Ile Val Ser Ile Leu Arg Ser Trp Asn Glu (SEQ ID NO: 14);
wherein the replaced amino acid residues are replaced by homologous amino acid residues.

3. The pharmaceutical composition according to claim 1 or 2, wherein the amino acid sequence X1-X14 is represented by any one of the following sequences:
a) Leu Leu Arg Ile Ser Leu Leu Leu Ile Gin Ser Trp Leu Glu (SEQ ID NO: 12);
b) Leu Leu Arg Ile Ser Leu Leu Leu Ile Glu Ser Trp Leu Glu (SEQ ID NO: 13);
c) Phe Leu Ser Leu Ile Val Ser Ile Leu Arg Ser Trp Asn Glu (SEQ ID NO: 14).

4. The pharmaceutical composition according to any one of claims 1 to 3, wherein the peptide of B) comprises or consists of an amino acid sequence selected from any 6mer, 7mer, 8mer, 9mer 10mer, 11mer, 12mer, 13mer fragment of the amino acid sequence selected from the group consisting of
a) the general formula (I):
X1-X2-X3-X4-X5-X6-X7-X8-X9-X10-X11-X12-X13-X14, wherein the amino acid residues X1 to X14 are as defined in claim 1;
b) Leu Leu Arg Ile Ser Leu Leu Leu Ile Gln Ser Trp Leu Glu (SEQ ID NO: 12);
c) Leu Leu Arg Ile Ser Leu Leu Leu Ile Glu Ser Trp Leu Glu (SEQ ID NO: 13);
d) Phe Leu Ser Leu Ile Val Ser Ile Leu Arg Ser Trp Asn Glu (SEQ ID NO: 14); or wherein the peptide comprises or consists of the respective 14mer amino acid sequence.

5. The pharmaceutical composition according to any one of claims 1 to 4, wherein the peptide of B) comprises or consists of an amino acid sequence selected from the following:
a) Leu Leu Arg Ile Ser Leu (SEQ ID NO: 15);
b) Arg Ile Ser Leu Leu Leu (SEQ ID NO: 16);
c) Ser Leu Leu Leu Ile Gln (SEQ ID NO: 17);
d) Leu Leu Ile Gln Ser Trp (SEQ ID NO: 18);
e) Ile Gln Ser Trp Leu Glu (SEQ ID NO: 19);
f) Ser Leu Leu Leu Ile Glu (SEQ ID NO: 20);
g) Leu Leu Ile Glu Ser Trp (SEQ ID NO: 21);
h) Ile Glu Ser Trp Leu Glu (SEQ ID NO: 22);
i) Phe Leu Ser Leu Ile Val (SEQ ID NO: 23);
j) Ser Leu Ile Val Ser Ile (SEQ ID NO: 24);
k) Ile Val Ser Ile Leu Arg (SEQ ID NO: 25);
l) Ser Ile Leu Arg Ser Trp (SEQ ID NO: 26);
m) Leu Arg Ser Trp Asn Glu (SEQ ID NO:27).

6. The use of a compound for the manufacture of a medicament for use in the preventive and/or therapeutic treatment of thrombosis-related diseases or conditions relating to high level of PAI-1 expression, which compound is selected from the following:
A) a fragment of a polypeptide/protein of prolactin (PRL)-growth hormone (GH) - placental lactogen (PL)-family and homologous derivatives thereof, which fragment comprises from 6 to 14 consecutive amino acid residues of the amino acid sequence having the following general formula (I):
X1-X2-X3-X4-X5-X6-X7-X8-X9-X10-X11-X12-X13-X14, wherein the amino acid residues X1 to X14 are as defined in claim 1;
B) a peptide or a recombinant protein comprising said peptide, wherein the peptide is from 6 to 50 amino acids in length and having the activity to inhibit plasminogen activator inhibitor I (PAI-1), said peptide comprising from 6 to 14 consecutive amino acid residues of the amino acid sequence of said general formula (I);
C) a polynucleotide encoding said fragment of a polypeptide or protein of A) or said peptide or recombinant protein of B).

7. A method for the preventive and/or therapeutic treatment of thrombosis-related diseases or conditions relating to high level of PAI-1 expression, comprising the administration of a pharmaceutically active amount of the compound as defined in claim 6.

8. The pharmaceutical composition, use or method according to any one of claims 1 to 7, wherein the thrombosis-related disease is selected from venous thrombosis, arterial thrombosis, acute myocardial infarction, stroke.

9. A peptide or a recombinant protein comprising said peptide, wherein the peptide is from 6 to 50 amino acids in length and comprises from 6 to 10 consecutive amino acid residues of the amino acid sequence of the amino acid sequence of the general formula (I):
X1-X2-X3-X4-X5-X6-X7-X8-X9-X10-X11-X12-X13-X14, wherein the amino acid residues X1 to X14 are as defined in claim 1;
with the proviso that said peptide or said recombinant protein does not comprise or consist of any one of the following amino acid sequences:
a) from 11 to 14 consecutive amino acid residues of the amino acid sequence of the amino acid sequence of the general formula (I);
b) wherein the amino acid sequence X1-X14 of the peptide is having at least 70% identity to any one of the following sequences:
i) Leu Leu Arg Ile Ser Leu Leu Leu Ile Gln Ser Trp Leu Glu (SEQ ID NO: 12);
ii) Leu Leu Arg Ile Ser Leu Leu Leu Ile Glu Ser Trp Leu Glu (SEQ ID NO: 13);
iii) Phe Leu Ser Leu Ile Val Ser Ile Leu Arg Ser Trp Asn Glu (SEQ ID NO: 14);
c) Leu Leu Ile Gln Ser Trp Leu Glu Pro Val Gln Phe Leu Arg Ser Val Phe Ala Asn Ser (SEQ ID NO: 28)

10. The peptide according to claim 9, wherein the peptide comprises or consists of an amino acid sequence selected from a 6mer, 7mer, 8mer, 9mer, 10mer fragment of the amino acid sequence of the general formula (I):
X1-X2-X3-X4-X5-X6-X7-X8-X9-X10-X11-X12-X13-X14,
wherein the amino acid residues X1 to X14 are as defined in claim 1.

11. The peptide according to claim 9 or 10, wherein the peptide comprises or consists of an amino acid sequence selected from the following:
a) Leu Leu Arg Ile Ser Leu (SEQ ID NO: 15);
b) Arg Ile Ser Leu Leu Leu (SEQ ID NO: 16);
c) Ser Leu Leu Leu Ile Gln (SEQ ID NO: 17);
d) Leu Leu Ile Gln Ser Trp (SEQ ID NO: 18);
e) Ile Gln Ser Trp Leu Glu (SEQ ID NO: 19);
f) Ser Leu Leu Leu Ile Glu (SEQ ID NO: 20);
g) Leu Leu Ile Glu Ser Trp (SEQ ID NO: 21);
h) Ile Glu Ser Trp Leu Glu (SEQ ID NO: 22);
i) Phe Leu Ser Leu Ile Val (SEQ ID NO: 23);
j) Ser Leu Ile Val Ser Ile (SEQ ID NO: 24);
k) Ile Val Ser Ile Leu Arg (SEQ ID NO: 25);
l) Ser Ile Leu Arg Ser Trp (SEQ ID NO: 26);
m) Leu Arg Ser Trp Asn Glu (SEQ ID NO:27).

12. A polynucleotide encoding the peptide or recombinant protein of any one of claims 9 to 11.

13. A transformant comprising the polynucleotide of claim 1 or 12.

14. A pharmaceutical composition comprising the peptide of any one of claims 9 to 11 or the polynucleotide of claim 12.


SEQUENCE LISTING

<110>  Université de Liège

<120>  A pharmaceutical composition for treatment of
thrombosis-related
       diseases comprising a fragment of prolactin (PRL)-growth
hormone
       (GH) - placental lactogen (PL)-family protein

<130>  P15209EP

<160>  36

<170>  PatentIn version 3.3

<210>  1
<211>  200
<212>  PRT
<213>  Artificial

<220>
<223>  synthetic peptide

<400>  1

Met Leu Pro Ile Cys Pro Gly Gly Ala Ala Arg Cys Gln Val Thr Leu
1               5                   10                  15

Arg Asp Leu Phe Asp Arg Ala Val Val Leu Ser His Tyr Ile His Asn
            20                  25                  30

Leu Ser Ser Glu Met Phe Ser Glu Phe Asp Lys Arg Tyr Thr His Gly
        35                  40                  45

Arg Gly Phe Ile Thr Lys Ala Ile Asn Ser Cys His Thr Ser Ser Leu
    50                  55                  60

Ala Thr Pro Glu Asp Lys Glu Gln Ala Gln Gln Met Asn Gln Lys Asp
65                  70                  75                  80

Phe Leu Ser Leu Ile Val Ser Ile Leu Arg Ser Trp Asn Glu Pro Leu
                85                  90                  95

Tyr His Leu Val Thr Glu Val Arg Gly Met Gln Glu Ala Pro Glu Ala
            100                 105                 110

Ile Leu Ser Lys Ala Val Glu Ile Glu Glu Gln Thr Lys Arg Leu Leu
        115                 120                 125

Glu Gly Met Glu Leu Ile Val Ser Gln Val His Pro Glu Thr Lys Glu
    130                 135                 140

Asn Glu Ile Tyr Pro Val Trp Ser Gly Leu Pro Ser Leu Gln Met Ala
145                 150                 155                 160

Asp Glu Glu Ser Arg Leu Ser Ala Tyr Tyr Asn Leu Leu His Cys Leu
                165                 170                 175

Arg Arg Asp Ser His Lys Ile Asp Asn Tyr Leu Lys Leu Leu Lys Cys
            180                 185                 190

Arg Ile Ile His Asn Asn Asn Cys
        195                 200

<210>  2
<211>  124
<212>  PRT
<213>  Artificial

<220>
<223>  synthetic peptide

<400>  2

Met Leu Pro Ile Cys Pro Gly Gly Ala Ala Arg Cys Gln Val Thr Leu
1               5                   10                  15

Arg Asp Leu Phe Asp Arg Ala Val Val Leu Ser His Tyr Ile His Asn
            20                  25                  30

Leu Ser Ser Glu Met Phe Ser Glu Phe Asp Lys Arg Tyr Thr His Gly
        35                  40                  45

Arg Gly Phe Ile Thr Lys Ala Ile Asn Ser Ser His Thr Ser Ser Leu
    50                  55                  60

Ala Thr Pro Glu Asp Lys Glu Gln Ala Gln Gln Met Asn Gln Lys Asp
65                  70                  75                  80

Phe Leu Ser Leu Ile Val Ser Ile Leu Arg Ser Trp Asn Glu Pro Leu
                85                  90                  95

Tyr His Leu Val Thr Glu Val Arg Gly Met Gln Glu Ala Pro Glu Ala
            100                 105                 110

Ile Leu Ser Lys Ala Val Glu Ile Glu Glu Gln Thr
        115                 120

<210>  3
<211>  140
<212>  PRT
<213>  Artificial

<220>
<223>  synthetic peptide

<400>  3

Met Leu Pro Ile Cys Pro Gly Gly Ala Ala Arg Cys Gln Val Thr Leu
1               5                   10                  15

Arg Asp Leu Phe Asp Arg Ala Val Val Leu Ser His Tyr Ile His Asn
            20                  25                  30

Leu Ser Ser Glu Met Phe Ser Glu Phe Asp Lys Arg Tyr Thr His Gly
        35                  40                  45

Arg Gly Phe Ile Thr Lys Ala Ile Asn Ser Ser His Thr Ser Ser Leu
    50                  55                  60

Ala Thr Pro Glu Asp Lys Glu Gln Ala Gln Gln Met Asn Gln Lys Asp
65                  70                  75                  80

Phe Leu Ser Leu Ile Val Ser Ile Leu Arg Ser Trp Asn Glu Pro Leu
                85                  90                  95

Tyr His Leu Val Thr Glu Val Arg Gly Met Gln Glu Ala Pro Glu Ala
            100                 105                 110

Ile Leu Ser Lys Ala Val Glu Ile Glu Glu Gln Thr Lys Arg Leu Leu

115                 120                 125

Glu Gly Met Glu Leu Ile Val Ser Gln Val His Pro
    130                 135                 140

<210>  4
<211>  143
<212>  PRT
<213>  Artificial

<220>
<223>  synthetic peptide

<400>  4

Met Leu Pro Ile Cys Pro Gly Gly Ala Ala Arg Cys Gln Val Thr Leu
1               5                   10                  15

Arg Asp Leu Phe Asp Arg Ala Val Val Leu Ser His Tyr Ile His Asn
            20                  25                  30

Leu Ser Ser Glu Met Phe Ser Glu Phe Asp Lys Arg Tyr Thr His Gly
        35                  40                  45

Arg Gly Phe Ile Thr Lys Ala Ile Asn Ser Ser His Thr Ser Ser Leu
    50                  55                  60

Ala Thr Pro Glu Asp Lys Glu Gln Ala Gln Gln Met Asn Gln Lys Asp
65                  70                  75                  80

Phe Leu Ser Leu Ile Val Ser Ile Leu Arg Ser Trp Asn Glu Pro Leu
                85                  90                  95

Tyr His Leu Val Thr Glu Val Arg Gly Met Gln Glu Ala Pro Glu Ala
            100                 105                 110

Ile Leu Ser Lys Ala Val Glu Ile Glu Glu Gln Thr Lys Arg Leu Leu
        115                 120                 125

Glu Gly Met Glu Leu Ile Val Ser Gln Val His Pro Arg Pro Pro
    130                 135                 140

<210>  5
<211>  192
<212>  PRT
<213>  Artificial

<220>
<223>  synthetic peptide

<400>  5

Met Val Gln Thr Val Pro Leu Ser Arg Leu Phe Asp His Ala Met Leu
1               5                   10                  15

Gln Ala His Arg Ala His Gln Leu Ala Ile Asp Thr Tyr Gln Glu Phe
            20                  25                  30

Glu Glu Thr Tyr Ile Pro Lys Asp Gln Lys Tyr Ser Phe Leu His Asp
        35                  40                  45

Ser Gln Thr Ser Phe Cys Phe Ser Asp Ser Ile Pro Thr Pro Ser Asn
    50                  55                  60

Met Glu Glu Thr Gln Gln Lys Ser Asn Leu Glu Leu Leu Arg Ile Ser
65                  70                  75                  80

Leu Leu Leu Ile Glu Ser Trp Leu Glu Pro Val Arg Phe Leu Arg Ser
                85                  90                  95

Met Phe Ala Asn Asn Leu Val Tyr Asp Thr Ser Asp Ser Asp Asp Tyr
            100                 105                 110

His Leu Leu Lys Asp Leu Glu Glu Gly Ile Gln Thr Leu Met Gly Arg
        115                 120                 125

Leu Glu Asp Gly Ser Arg Arg Thr Gly Gln Ile Leu Lys Gln Thr Tyr
    130                 135                 140

Ser Lys Phe Asp Thr Asn Ser His Asn His Asp Ala Leu Leu Lys Asn
145                 150                 155                 160

Tyr Gly Leu Leu Tyr Cys Phe Arg Lys Asp Met Asp Lys Val Glu Thr
                165                 170                 175

Phe Leu Arg Met Val Gln Cys Arg Ser Val Glu Gly Ser Cys Gly Phe
            180                 185                 190

<210>  6
<211>  135
<212>  PRT
<213>  Artificial

<220>
<223>  synthetic peptide

<400>  6

Met Val Gln Thr Val Pro Leu Ser Arg Leu Phe Asp His Ala Met Leu
1               5                   10                  15

Gln Ala His Arg Ala His Gln Leu Ala Ile Asp Thr Tyr Gln Glu Phe
            20                  25                  30

Glu Glu Thr Tyr Ile Pro Lys Asp Gln Lys Tyr Ser Phe Leu His Asp
        35                  40                  45

Ser Gln Thr Ser Phe Ser Phe Ser Asp Ser Ile Pro Thr Pro Ser Asn
    50                  55                  60

Met Glu Glu Thr Gln Gln Lys Ser Asn Leu Glu Leu Leu Arg Ile Ser
65                  70                  75                  80

Leu Leu Leu Ile Glu Ser Trp Leu Glu Pro Val Arg Phe Leu Arg Ser
                85                  90                  95

Met Phe Ala Asn Asn Leu Val Tyr Asp Thr Ser Asp Ser Asp Asp Tyr
            100                 105                 110

His Leu Leu Lys Asp Leu Glu Glu Gly Ile Gln Thr Leu Met Gly Arg
        115                 120                 125

Leu Glu Asp Gly Ser Pro Arg
    130                 135

<210>  7
<211>  192

<212>  PRT
<213>  Artificial

<220>
<223>  synthetic peptide

<400>  7

Met Phe Pro Thr Ile Pro Leu Ser Arg Leu Phe Asp Asn Ala Met Leu
1               5                   10                  15

Arg Ala His Arg Leu His Gln Leu Ala Phe Asp Thr Tyr Gln Glu Phe
            20                  25                  30

Glu Glu Ala Tyr Ile Pro Lys Glu Gln Lys Tyr Ser Phe Leu Gln Asn
        35                  40                  45

Pro Gln Thr Ser Leu Cys Phe Ser Glu Ser Ile Pro Thr Pro Ser Asn
    50                  55                  60

Arg Glu Glu Thr Gln Gln Lys Ser Asn Leu Glu Leu Leu Arg Ile Ser
65                  70                  75                  80

Leu Leu Leu Ile Gln Ser Trp Leu Glu Pro Val Gln Phe Leu Arg Ser
                85                  90                  95

Val Phe Ala Asn Ser Leu Val Tyr Gly Ala Ser Asp Ser Asn Val Tyr
            100                 105                 110

Asp Leu Leu Lys Asp Leu Glu Glu Gly Ile Gln Thr Leu Met Gly Arg
        115                 120                 125

Leu Glu Asp Gly Ser Pro Arg Thr Gly Gln Ile Phe Lys Gln Thr Tyr
    130                 135                 140

Ser Lys Phe Asp Thr Asn Ser His Asn Asp Asp Ala Leu Leu Lys Asn
145                 150                 155                 160

Tyr Gly Leu Leu Tyr Cys Phe Arg Lys Asp Met Asp Lys Val Glu Thr
                165                 170                 175

Phe Leu Arg Ile Val Gln Cys Arg Ser Val Glu Gly Ser Cys Gly Phe

180                 185                 190

<210>  8
<211>  134
<212>  PRT
<213>  Artificial

<220>
<223>  synthetic peptide

<400>  8

Met Phe Pro Thr Ile Pro Leu Ser Arg Leu Phe Asp Asn Ala Met Leu
1               5                   10                  15

Arg Ala His Arg Leu His Gln Leu Ala Phe Asp Thr Tyr Gln Glu Phe
            20                  25                  30

Glu Glu Ala Tyr Ile Pro Lys Glu Gln Lys Tyr Ser Phe Leu Gln Asn
        35                  40                  45

Pro Gln Thr Ser Leu Ser Phe Ser Glu Ser Ile Pro Thr Pro Ser Asn
    50                  55                  60

Arg Glu Glu Thr Gln Gln Lys Ser Asn Leu Glu Leu Leu Arg Ile Ser
65                  70                  75                  80

Leu Leu Leu Ile Gln Ser Trp Leu Glu Pro Val Gln Phe Leu Arg Ser
                85                  90                  95

Val Phe Ala Asn Ser Leu Val Tyr Gly Ala Ser Asp Ser Asn Val Tyr
            100                 105                 110

Asp Leu Leu Lys Asp Leu Glu Glu Gly Ile Gln Thr Leu Met Gly Arg
        115                 120                 125

Leu Glu Asp Gly Ser Pro
    130

<210>  9
<211>  192
<212>  PRT
<213>  Artificial

<220>
<223>  synthetic peptide

<400>  9

Met Phe Pro Thr Ile Pro Leu Ser Arg Leu Phe Asp Asn Ala Met Leu
1               5                   10                  15

Arg Ala Arg Arg Leu Tyr Gln Leu Ala Tyr Asp Thr Tyr Gln Glu Phe
            20                  25                  30

Glu Glu Ala Tyr Ile Leu Lys Glu Gln Lys Tyr Ser Phe Leu Gln Asn
        35                  40                  45

Pro Gln Thr Ser Leu Cys Phe Ser Glu Ser Ile Pro Thr Pro Ser Asn
    50                  55                  60

Arg Val Lys Thr Gln Gln Lys Ser Asn Leu Glu Leu Leu Arg Ile Ser
65                  70                  75                  80

Leu Leu Leu Ile Gln Ser Trp Leu Glu Pro Val Gln Leu Leu Arg Ser
                85                  90                  95

Val Phe Ala Asn Ser Leu Val Tyr Gly Ala Ser Asp Ser Asn Val Tyr
            100                 105                 110

Arg His Leu Lys Asp Leu Glu Glu Gly Ile Gln Thr Leu Met Trp Arg
        115                 120                 125

Leu Glu Asp Gly Ser Pro Arg Thr Gly Gln Ile Phe Asn Gln Ser Tyr
    130                 135                 140

Ser Lys Phe Asp Thr Lys Ser His Asn Asp Asp Ala Leu Leu Lys Asn
145                 150                 155                 160

Tyr Gly Leu Leu Tyr Cys Phe Arg Lys Asp Met Asp Lys Val Glu Thr
                165                 170                 175

Phe Leu Arg Ile Val Gln Cys Arg Ser Val Glu Gly Ser Cys Gly Phe
            180                 185                 190

<210>  10
<211>  135
<212>  PRT
<213>  Artificial

<220>
<223>  synthetic peptide

<400>  10

Met Phe Pro Thr Ile Pro Leu Ser Arg Leu Phe Asp Asn Ala Met Leu
1               5                   10                  15

Arg Ala Arg Arg Leu Tyr Gln Leu Ala Tyr Asp Thr Tyr Gln Glu Phe
            20                  25                  30

Glu Glu Ala Tyr Ile Leu Lys Glu Gln Lys Tyr Ser Phe Leu Gln Asn
        35                  40                  45

Pro Gln Thr Ser Leu Cys Phe Ser Glu Ser Ile Pro Thr Pro Ser Asn
    50                  55                  60

Arg Val Lys Thr Gln Gln Lys Ser Asn Leu Glu Leu Leu Arg Ile Ser
65                  70                  75                  80

Leu Leu Leu Ile Gln Ser Trp Leu Glu Pro Val Gln Leu Leu Arg Ser
                85                  90                  95

Val Phe Ala Asn Ser Leu Val Tyr Gly Ala Ser Asp Ser Asn Val Tyr
            100                 105                 110

Arg His Leu Lys Asp Leu Glu Glu Gly Ile Gln Thr Leu Met Trp Arg
        115                 120                 125

Leu Glu Asp Gly Ser Pro Arg
    130                 135

<210>  11
<211>  14
<212>  PRT
<213>  Artificial

<220>

<223>  synthetic peptide

<220>
<221>  VARIANT
<222>  (1)..(1)
<223>  Leu, Phe

<220>
<221>  VARIANT
<222>  (3)..(3)
<223>  Arg, Ser

<220>
<221>  VARIANT
<222>  (4)..(4)
<223>  Ile, Leu

<220>
<221>  VARIANT
<222>  (5)..(5)
<223>  Ser, Ile

<220>
<221>  VARIANT
<222>  (6)..(6)
<223>  Leu, Val

<220>
<221>  VARIANT
<222>  (7)..(7)
<223>  Leu, Ser

<220>
<221>  VARIANT
<222>  (8)..(8)
<223>  Leu, Ile

<220>
<221>  VARIANT
<222>  (9)..(9)
<223>  Ile, Leu

<220>
<221>  VARIANT
<222>  (10)..(10)
<223>  Gln, Glu, Arg

<220>
<221>  VARIANT
<222>  (13)..(13)
<223>  Leu, Asn

<400>  11

Xaa Leu Xaa Xaa Xaa Xaa Xaa Xaa Xaa Xaa Ser Trp Xaa Glu
1               5                   10


<210>  12
<211>  14
<212>  PRT
<213>  Artificial

<220>
<223>  synthetic peptide

<400>  12

Leu Leu Arg Ile Ser Leu Leu Leu Ile Gln Ser Trp Leu Glu
1               5                   10


<210>  13
<211>  14
<212>  PRT
<213>  Artificial

<220>
<223>  synthetic peptide

<400>  13

Leu Leu Arg Ile Ser Leu Leu Leu Ile Glu Ser Trp Leu Glu
1               5                   10


<210>  14
<211>  14
<212>  PRT
<213>  Artificial

<220>
<223>  synthetic peptide

<400>  14

Phe Leu Ser Leu Ile Val Ser Ile Leu Arg Ser Trp Asn Glu
1               5                   10


<210>  15
<211>  6
<212>  PRT
<213>  Artificial

<220>
<223>  synthetic peptide

<400>  15

Leu Leu Arg Ile Ser Leu
1               5


<210>  16
<211>  6
<212>  PRT
<213>  Artificial

<220>
<223>  synthetic peptide

<400>  16

Arg Ile Ser Leu Leu Leu
1               5


<210>  17
<211>  6
<212>  PRT
<213>  Artificial

<220>
<223>  synthetic peptide

<400>  17

Ser Leu Leu Leu Ile Gln
1               5


<210>  18
<211>  6
<212>  PRT
<213>  Artificial

<220>
<223>  synthetic peptide

<400>  18

Leu Leu Ile Gln Ser Trp
1               5


<210>  19
<211>  6

<212>  PRT
<213>  Artificial

<220>
<223>  synthetic peptide

<400>  19

Ile Gln Ser Trp Leu Glu
1               5


<210>  20
<211>  6
<212>  PRT
<213>  Artificial

<220>
<223>  synthetic peptide

<400>  20

Ser Leu Leu Leu Ile Glu
1               5


<210>  21
<211>  6
<212>  PRT
<213>  Artificial

<220>
<223>  synthetic peptide

<400>  21

Leu Leu Ile Glu Ser Trp
1               5


<210>  22
<211>  6
<212>  PRT
<213>  Artificial

<220>
<223>  synthetic peptide

<400>  22

Ile Glu Ser Trp Leu Glu
1               5

<210>  23
<211>  6
<212>  PRT
<213>  Artificial

<220>
<223>  synthetic peptide

<400>  23

Phe Leu Ser Leu Ile Val
1               5


<210>  24
<211>  6
<212>  PRT
<213>  Artificial

<220>
<223>  synthetic peptide

<400>  24

Ser Leu Ile Val Ser Ile
1               5


<210>  25
<211>  6
<212>  PRT
<213>  Artificial

<220>
<223>  synthetic peptide

<400>  25

Ile Val Ser Ile Leu Arg
1               5


<210>  26
<211>  6
<212>  PRT
<213>  Artificial

<220>
<223>  synthetic peptide

<400>  26

Ser Ile Leu Arg Ser Trp
1               5

<210>  27
<211>  6
<212>  PRT
<213>  Artificial

<220>
<223>  synthetic peptide

<400>  27

Leu Arg Ser Trp Asn Glu
1               5

<210>  28
<211>  20
<212>  PRT
<213>  Artificial

<220>
<223>  synthetic peptide

<400>  28

Leu Leu Ile Gln Ser Trp Leu Glu Pro Val Gln Phe Leu Arg Ser Val
1               5                   10                  15

Phe Ala Asn Ser
            20

<210>  29
<211>  20
<212>  PRT
<213>  Artificial

<220>
<223>  synthetic peptide

<400>  29

Ser Asn Leu Glu Leu Leu Arg Ile Ser Leu Leu Leu Ile Gln Ser Trp
1               5                   10                  15

Leu Glu Pro Val
            20

<210>  30
<211>  37
<212>  PRT
<213>  Artificial

<220>
<223>  synthetic peptide

<400>  30

Asn Gln Lys Asp Phe Leu Ser Leu Ile Val Ser Ile Leu Arg Ser Trp
1               5                   10                  15

Asn Glu Pro Leu Tyr His Leu Val Thr Glu Val Arg Gly Met Gln Glu
            20                  25                  30

Ala Pro Glu Ala Ile
        35

<210>  31
<211>  29
<212>  PRT
<213>  Artificial

<220>
<223>  synthetic peptide

<400>  31

Asn Leu Glu Leu Leu Arg Ile Ser Leu Leu Leu Ile Gln Ser Trp Leu
1               5                   10                  15

Glu Pro Val Gln Phe Leu Arg Ser Val Phe Ala Asn Ser
            20                  25

<210>  32
<211>  14
<212>  PRT
<213>  Artificial

<220>
<223>  synthetic peptide

<400>  32

Leu Ser Gln Ile Leu Ser Ser Leu Ile Gln Ser Trp Leu Glu
1               5                   10

<210>  33
<211>  14
<212>  PRT
<213>  Artificial

<220>
<223>  synthetic peptide

<400>  33

Phe Asn Ser Leu Ile Ser Ser Ile Leu Arg Val Trp Leu Glu
1               5                   10


<210>  34
<211>  17
<212>  PRT
<213>  Artificial

<220>
<223>  synthetic peptide

<400>  34

Ile Ser Leu Leu Leu Ile Gln Ser Trp Leu Glu Pro Val Gln Phe Leu
1               5                   10                  15


Arg




<210>  35
<211>  25
<212>  RNA
<213>  Artificial

<220>
<223>  synthetic RNA

<400>  35
ccgauuuacu gaagaauugc acaga
   25


<210>  36
<211>  25
<212>  RNA
<213>  Artificial

<220>

<223>  synthetic RNA

<400>  36
gaauguaacc uaauagaacc cuaau
   25